# EUROPEAN PATENT SPECIFICATION

(11) **EP 2 859 100 B1**
(45) Date of publication and mention of the grant of the patent: **30.08.2017**
(21) Application number: 13724630.2
(22) Date of filing: 12.04.2013
(51) Int. Cl.: C12N 15/10

(54) **AN EXPRESSION VECTOR CONTAINING STRONG PROMOTER USEFUL FOR HIGH LEVEL EXPRESSION OF HETEROLOGOUS GENES IN SCHIZOSACCHAROMYCES POMBE AND METHOD FOR PRODUCTION OF DESIRED PROTEINS THEREOF**
EXPRESSIONSVEKTOR MIT EINEM STARKEN PROMOTOR FÜR HOHE EXPRESSION HETEROLOGER GENE IN SCHIZOSACCHAROMYCES POMBE UND VERFAHREN ZUR HERSTELLUNG GEWÜNSCHTER PROTEINE DARAUS
VECTEUR D'EXPRESSION CONTENANT UN PROMOTEUR FORT UTILE POUR L'EXPRESSION À HAUT NIVEAU DE GÈNES HÉTÉROLOGUES DANS SCHIZOSACCHAROMYCES POMBE ET PROCÉDÉ DE PRODUCTION DE PROTÉINES SOUHAITÉES PAR L'UTILISATION DE CELUI-CI

(30) Priority: 11.06.2012 IN 1788DE2012
(43) Date of publication of application: 15.04.2015
(73) Proprietor: Council of Scientific and Industrial Research, New Delhi- 110001 (IN)
(72) Inventor: SINGH, Jagmohan, Chandigarh 160 036 (IN); VERMA, Hemant Kumar, Chandigarh 160 036 (IN)
(74) Representative: Cabinet Plasseraud
(86) International application number: PCT/IN2013/000247
(87) International publication number: WO 2013/186788

(56) References cited:
- US-A- 5 663 061
- Verma, Hemant Kumar: "Development of expression system for expression of therapeutic proteins in fission yeast schizosaccharomyces pombe.", Digital Repository of publications CSIR Insitute of Microbial Technology , 10 June 2012 (2012-06-10), XP002716735, Retrieved from the Internet: URL:http://crdd.osdd.net/open/1485/1/TH-26 7.pdf [retrieved on 2013-11-14]
- YOKOYAMA KAZUAKI ET AL: "Expression of a novel 90-kDa protein, Lsd90, involved in the metabolism of very long-chain fatty acid-containing phospholipids in a mitosis-defective fission yeast mutant", JOURNAL OF BIOCHEMISTRY, vol. 143, no. 3, March 2008 (2008-03), pages 369-375, XP002716736, (TOKYO) ISSN: 0021-924X
- FORSBURG SUSAN L: "Comparison of Schizosaccharomyces pombe expression systems", NUCLEIC ACIDS RESEARCH, vol. 21, no. 12, 1993, pages 2955-2956, XP002716737, ISSN: 0305-1048

## Description

### FIELD OF THE INVENTION

The present invention relates to an expression vector containing a strong promoter for high level heterologous gene expression in fission yeast *Schizosaccharomyces pombe* and method for production of desired protein thereof.

More particularly, the present invention relates to super-strength, sorbitol-stimulated promoter of the *lsd90* gene of *Schizosaccharomyces pombe* which is selectively introduced in an expression vector to get high level heterologous gene expression in *S. pombe.*

The main use of this promoter is high level of protein expression especially of therapeutic interest from higher eukaryotes including humans.

### BACKGROUND OF THE INVENTION

Since there are large number of similarities with higher eukaryotes, including humans, fission yeast *Schizosaccharomyces pombe* (hereinafter referred as *S*. *pombe*) is thought to be a potentially ideal host for expressing human proteins of therapeutic and industrial importance {review by Giga-Hama, 1997, in Foreign Gene Expression in Fission Yeast Schizosaccharomyces pombe (Giga-Hama and Kumagai, Eds.), Springer-Verlag, Berlin pp. 3-28}. However, this potential is yet to be realized because of the lack of user-friendly vectors that could be exploited for developing cost-effective process for expression of proteins.

Among the well-characterized yeast species, *S. pombe* is considered an attractive host for the production of heterologous proteins with complicated structures, such as glycosylated proteins derived from higher animals (Takegawa et al., 2009, Biotechnol. Appl. Biochem. 53: 227-235). N-terminal acetylation occurs in a similar way to those in mammalian cells. Proteins that are acetylated in mammalian cells might also be expected to be acetylated in yeast cells. Indeed, human lipocortin 1, which is an N-acetylated protein, was expressed in *S. pombe* with its acetylated N-terminal (Giga-Hama et al., 1994, Bio/Technology 12: 400-404). Golgi apparatus in *S. pombe* is well organized and morphologically distinct, whereas *S*. *cerevisiae* has golgi with poor morphological distinctions (Smith and Svoboda, 1972, Microbios 5: 177-182). *S. pombe* has also been reported to be a suitable host for the secretion of heterologous proteins. The secretory signal peptides P-factor and Cpy1, a vacuolar CPY (carboxypeptidase Y) have been used for efficient secretion of heterologous proteins (Ikeda et al., 2004, Biosci. Bioengi. 80: 366-373; Kjaerulff and Jensen, 2005, Biochem. Biophys. Res. Comm. 336: 9974-982). These large numbers of similarities have enhanced the desirability of exploring the possibility of *S. pombe* as an expression system.

The transcription-initiation mechanism of *S. pombe* is more similar to that of higher eukaryotes than of *Saccharomyces cerevisiae* (Russell, 1983, Nature 301: 167-169). The *S. pombe* TATA element is located 25-30 bp upstream of the start of transcription, exactly as in mammalian cells (Benoist et al., 1980, Nucleic Acids Res. 8: 127-142). Moreover it has been found that promoter melting and initiation in fission yeast is much more similar to humans than to budding yeast (Wai et al., 2004, J. Mol. Biol. 340: 981-989). Furthermore, the intron splicing machinery is very similar between the fission yeast and higher eukaryotes; human genes containing introns can be spliced in *S. pombe* but not in *S. cerevisiae* {Guthrie et al., 1986, (In Hicks, J., eds.), Yeast Cell Biology New York, pp. 301-321; Brennwald et al., 1988, Mol. Cell. Biol. 8: 5575-5580}. In addition, other similarities between *S*. *pombe* and higher eukaryotes include greater complexity of replication origins (Okuno et al., 1997, Nucleic Acids Res. 25: 530-536) and fission yeast centromeres, highly specialized chromosomal structures that resembles the centromeres of higher eukaryotes (Takahashi et al., 1992, Mol. Biol. Cell 3: 819-835). *S. pombe* has RNAi machinery genes like those in vertebrates, while these are lacking in *S. cerevisiae* (Schramke and Allshire, 2003, Science 301: 1069-1074). *S. cerevisiae* also has greatly simplified heterochromatin compared to *S. pombe.* In fact a high degree of sequence and chromatin organizational similarity of fission yeast with metazoans systems makes *S. pombe* an ideal model eukaryotic organism (Review by Niall Dillon, 2004, Heterochromatin structure and function in Biology of the Cell 96: 631-637). The similarities extend beyond the nuclear architecture. The signal-transduction system of *S*. *pombe* shows marked similarities to the mammalian G-protein-coupled system. Signals initiated by the mating factors of *S*. *pombe* are also transmitted through a G-protein to effector(s) (Xu et al., 1994, Mol. Cell. Biol. 14: 50-58).

Some viral and human promoters have also been found to functional in *S. pombe.* For example, *SV40* early promoter, human cytomegalovirus (*hCMV*) and human chorionic gonadotrophin □(*hCG*□*)* promoters: the latter two are nearly 10-fold stronger than the *SV40* promoter (Toyama, and Okayama, 1990, FEBS Lett. 268: 217-221). Indeed, successful expression has been achieved with *hCMV* promoter (Tohda et al., 1994, Gene 150: 275-280). Recently, two new promoters have been reported by Giga-Hama (Fujita et al., 2006, FEMS Yeast Res. 6: 883-887) and Bahler laboratories (Watt et al., 2008, Plos One 1: e1428). However, these are also unable to achieve high levels of expression.

The more well known strong promoters of *S*. *pombe* include the thiamine-regulated promoter *nmt1* (Maundrell, 1990, J. Biol. Chem. 265: 10857-64) and the constitutive promoter *adh1* (Russell, 1989, In: Molecular Biology of the Fission Yeast. San Diego: Academic Press, Inc. Nasim. A., Young, P., and Johnson, B. F., Eds. pp. 243-71). Thiamine acts as a small molecule repressor of the *nmt1* promoter. For regulated expression of proteins, genes of interest are cloned under the control of *nmt1* promoter and transformed into suitable strains of *S. pombe.* Cells harboring the vector are grown in the presence of the repressor thiamine. For induction, cells are washed with medium lacking thiamine and grown further for 15-18 hours in the same medium. The *adh1* is a strongest known constitutive promoter in *S. pombe* and can yield levels of protein to the extent of 0.5-2% of total cellular protein (Russell, 1989, In Molecular Biology of the Fission Yeast. San Diego: Academic Press, Inc. Nasim. A., Young, P., and Johnson, B. F., Eds. pp. 143-271).

A comparative study of the promoter strength has shown the *nmt1* promoter to be stronger than *adh1* (Forsburg, 1993, Nucleic Acids Res. 21: 2955-2956). Two derivatives of *nmt1, nmt41* and *nmt81,* provide intermediate to low level of expression of □-galactosidase (Forsburg, 1993, Nucleic Acids Res. 21: 2955-2956) and are used for physiological studies but are not suited for commercial scale expression.

Recently Kazuaki et al. reported a 90-Kda protein, Lsd90, involved in the metabolism of very long chain fatty acids in fission yeast mutant, which can be over expressed with an expression vector pREP81 having *nmt1* promoter (Kazuaki Yakoyama et al 2008, J. Biochem. 143:369-375).

Very recently, we have constructed a very high copy ATG vector exploiting the ability of a truncated copy of *URA3* gene of *S. cerevisiae* (*URA3m*) to impart high copy number under selection conditions and the *mat2P-RF* region linked to the mat2P locus in *S. pombe* that provides high transformation efficiency and plasmid stability to yield a vector that exists at ∼200 copies/cell, the highest copy number reported for any vector in *S. pombe* so far. In addition this vector we have incorporated the Ndel restriction site in the polylinker sequence that is useful for providing the first ATG codon for a direct cloning of any gene of interest for expression purpose (Verma and Singh, manuscript under preparation). However selective incorporation of 1.0 Kb upstream region of *lsd90* gene, as a promoter in any expression vector having ability to express heterologous gene, especially of therapeutic interest from higher eukaryotes including humans, is not reported earlier.

### BRIEF DESCRIPTION OF THE DRAWINGS

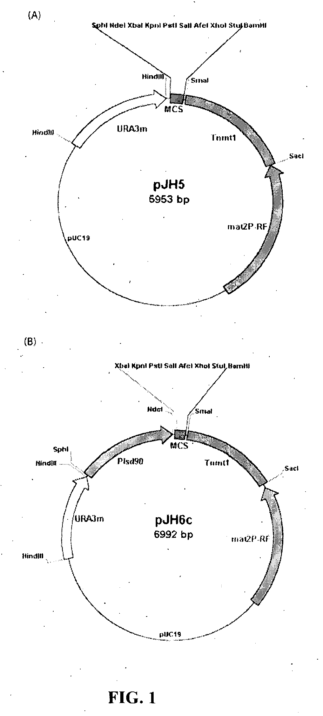

**Fig. 1** **Construction of a new expression vector in *S. pombe***
   (A) pUC19-based general-purpose ATG vector pJH5 in *S. pombe.* The vector having an Ndel site in its polylinker to provide ATG site for expression, also carries a severely truncated form of *URA3* gene, *URA3m, of S*. *cerevisiae* as selection marker, *mat2P*-right flank region (*mat2P-RF*) of *S. pombe* as an autonomous replicating sequence (ARS), a polylinker with wider choice of restriction sites and the transcription termination signal of the *nmt1* gene, *Tnmt1,* for efficient transcription termination and polyadenylation of the mRNA of the foreign gene to be expressed under a promoter. (B) Approximately 1.0 Kb upstream region *of S. pombe* gene *SPBC16E9.16c*/*lsd90* was PCR amplified and inserted into plasmid pJH5 at SphI/NdeI sites as a putative promoter *lsd90.* The resulting vector was designated pJH6c (Accession No. MTCC 5688).

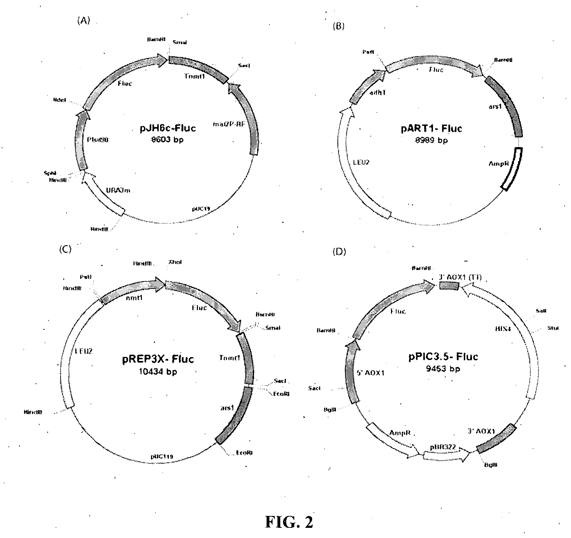

**Fig. 2** **Construction of *Fluc*-expression vectors**
   (A) pJH6c-Fluc vector containing *Fluc* gene cloned at Ndel and BamHI sites downstream of the new constitutive promoter *lsd90 of S. pombe.* (B) pART1-Fluc vector containing *Fluc* gene cloned at PstI and BamHI sites downstream of the constitutive promoter *adh1* of *S. pombe.* (C) pREP3X-Fluc vector containing *Fluc* gene cloned at Xhol and BamHI sites downstream of the thiamine-regulatable promoter *nmt1* of *S*. *pombe.* (D) pPIC3.5-Fluc vector of *P. pastoris* containing *Fluc* gene cloned at BamHI site downstream of the *AOX1* promoter.

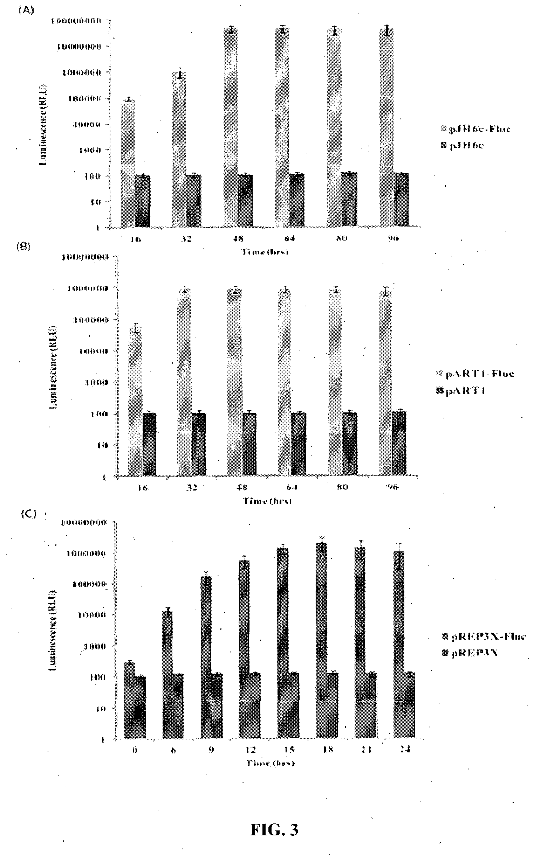

**Fig. 3** **Kinetics of luciferase expression under the control of constitutive promoters (*lsd90* and *adh1*) and thiamine-repressible promoter *nmt1* in *S*. *pombe***
   Cell extracts of cultures of strain *SPJ25* harbouring the plasmid construct (A) pJH6c-Fluc (*Fluc* gene under the control of *lsd90* promoter), (B) pART1-Fluc (*Fluc* gene under the control of *adh1* promoter) and (C) pREP3X-Fluc (*Fluc* gene under the control of *nmt1* promoter) were grown in selective media (PMA ura⁻ or PMA leu⁻) were subjected to measurement of luciferase activity.
**Fig. 4** **Kinetics of luciferase expression under the *AOX1* promoter of *P. pastoris*** Extracts of cultures of recombinant *GS115* strain harboring the gene *Fluc,* cloned under the control of *AOX1* promoter (pPIC3.5-Fluc), were assayed for luciferase activity.
**Fig. 5** **Growth kinetics of cultures harbouring the luciferase expression vectors** Cultures of cells of wild type strain expressing (A) pJH6c-Fluc and control vector pJH6c (Accession No. MTCC 5688) (B) pART1-Fluc and control vector pART1 (C) pREP3X-Fluc and control vector pREP3X were grown up to 48 hours. OD₆₀₀ was measured at indicated time points and plotted.
**Fig. 6** **Plasmid maps of *GFP*-expression vectors with *lsd90* (A), *adh1* (B) and *nmt1* (C) promoters**
   (A)Vector pJH6c-GFP containing *GFP* gene downstream of promoter *lsd90* at Sall and BamHI sites in the vector pJH6c (Accession No. MTCC 5688). (B) pART1-GFP vector containing *GFP* gene, which was cloned at PstI and BamHI sites downstream of the constitutive promoter *adh1* of S. *pombe* in the vector pART1. (C) pREP3X-GFP vector containing *GFP* gene, which was cloned at Xhol and BamHI sites downstream of the thiamine-regulatable promoter *nmt1* of S. *pombe* in the vector pREP3X.

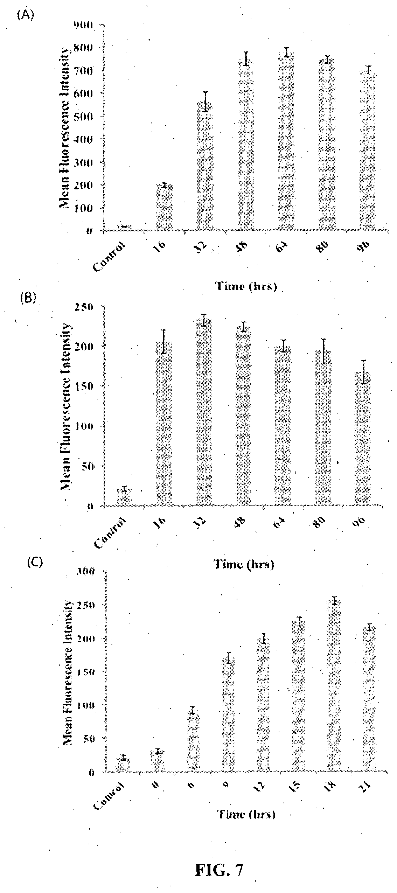

**Fig. 7** **Time-course of GFP expression under the control of *lsd90* (A), *adh1* (B) and *nmt1* (C) promoters**
   Graphical representation of FACS data showing the Mean Fluorescence Intensity (MFI) detected in cells expressing GFP with different vectors at indicated time points.

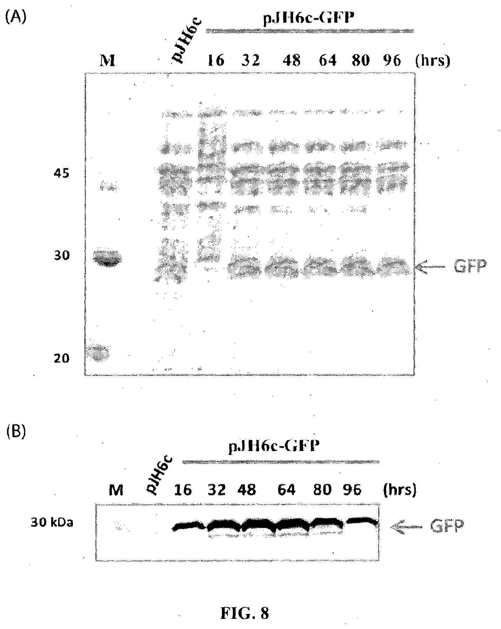

**Fig. 8** **Time course of GFP expression in wild-type strain of *S. pombe* detected by Coomassie staining (A) and Western blotting (B)**
   Whole cell extracts from cells expressing pJH6c-GFP or empty vector pJH6c (Accession No. MTCC 5688) vector alone. Cell extracts were subjected to SDS-polyacrylamide gel (12%) electrophoresis followed by, (A) staining with Coomassie blue, or (B) Western blotting using anti-GFP polyclonal antibody. M: Molecular Weight markers. GFP band of ∼28 kDa is indicated by arrow.

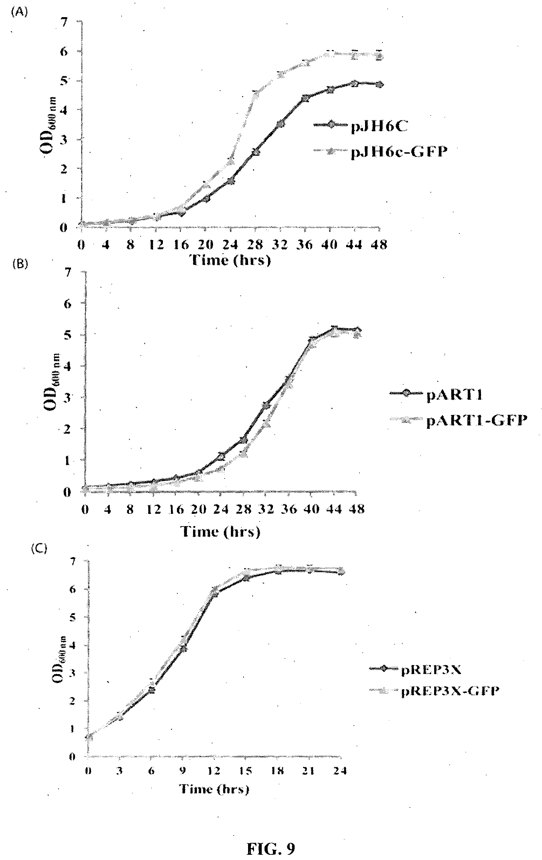

**Fig. 9** **Growth kinetics of the cultures harbouring *GFP* expression vector pJH6c-GFP and the control vector pJH6c (A), pART1-GFP and control vector pART1 (B), and pREP3X-GFP and control vector pREP3X (C)**
   Overnight cultures in selective medium were inoculated into the fresh medium at OD₆₀₀ 0.1 and grown further. Mean OD₆₀₀ values were plotted against time points of culture growth in duplicate up to early stationary phase.

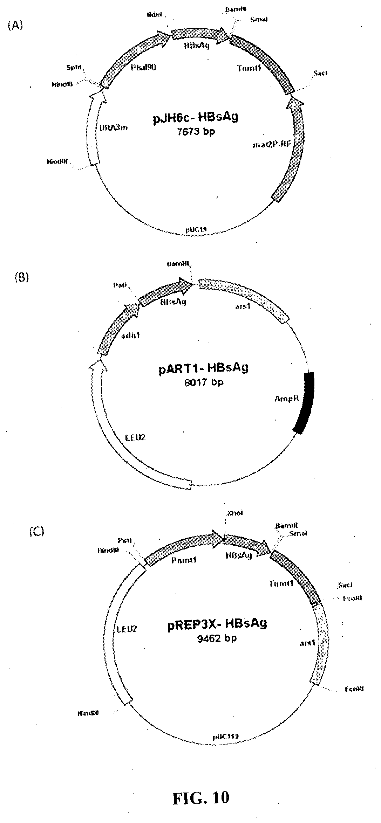

**Fig. 10** **Construction of vectors for *HBsAg* expression in *S. pombe***
   (A) pJH6c-HBsAg vector, containing *HBsAg* gene, cloned at Ndel and BamHI sites downstream of the new constitutive promoter *lsd90 of S. pombe* in the vector pJH6c (Accession No. MTCC 5688). (B) pARTI-HBsAg vector, containing *HBsAg* gene, cloned at PstI and BamHI sites downstream of the constitutive promoter *adh1* of *S. pombe* in vector pART1. (C) pREP3X-HBsAg vector, containing *HBsAg* gene, cloned at Xhol and BamHI sites downstream of the thiamine-regulatable promoter *nmt1* of S. *pombe* in vector pREP3X.

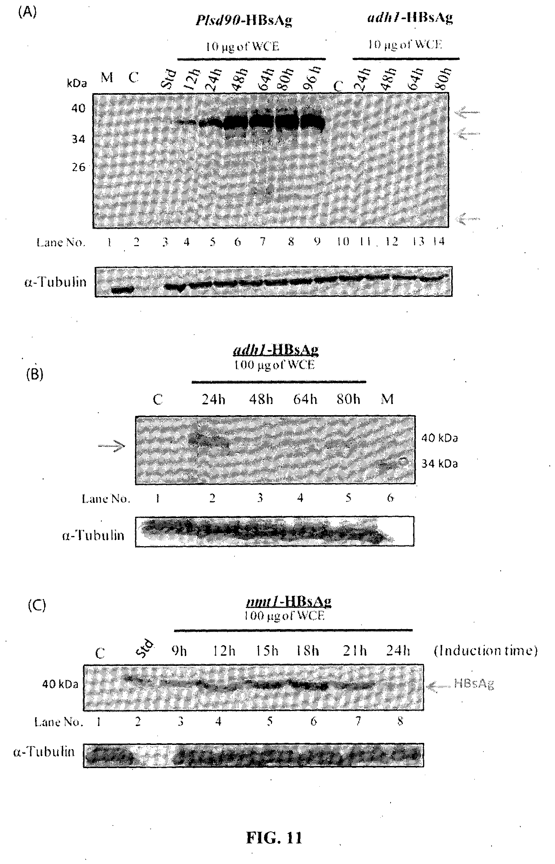

**Fig 11** **Time course of HBsAg expression under control of the constitutive promoters *adh1* and *lsd90* (A and B) and *nmt1* promoter (C) of *S*. *pombe***
   (A) Western blots of 10 µg of whole cell extracts (WCE) of cells harbouring control vectors (pJH6c and pART1) and vector expressing HBsAg under the control of promoters *lsd90* (plasmid pJH6c-HBsAg) and *adh1* (pART1-HBsAg), at indicated time points using anti-HBsAg polyclonal antibody. Lanes 2 and 10 represent vector controls pJH6c (Accession No. MTCC 5688) and pART1, respectively; lane 3, purified recombinant HBsAg standard (10 ng). M represents molecular weight protein marker. Arrows indicate the different oligomeric species of HBsAg.
   (B) A higher amount (100 µg) of cell extract prepared from the indicated cells was used for immunoblotting. Lane 1 represents the vector control pART1 and M, protein molecular weight markers.
   (C) 100 µg protein extract of vector control (lane 1) and recombinant vector pREP3X-HBsAg (lane 3-8) were subjected to immunoblotting with anti-HBsAg polyclonal antibody. Purified recombinant HBsAg protein (100 ng) from *P. pastoris* served as standard (lane 2).
   (A-C) The lower panels show the loading control α-tubulin.

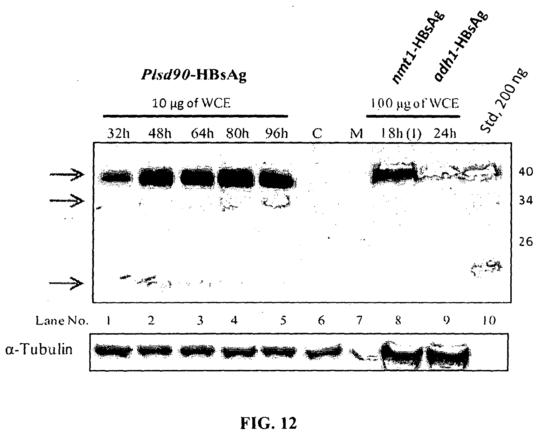

**Fig. 12** **Comparison of the level of expression of HBsAg achieved with different expression systems**
   Indicated amounts of whole cell extracts (WCE) of cells expressing HBsAg under different promoters of *S. pombe* for the indicated time periods, were subjected to Western blotting with anti-HBsAg polyclonal antibody. Lane 10 shows purified recombinant HBsAg protein (200 ng) as the standard and M represents protein size markers. Arrows indicate different oligomeric forms of HBsAg. The lower panel shows the loading control (α-tubulin).

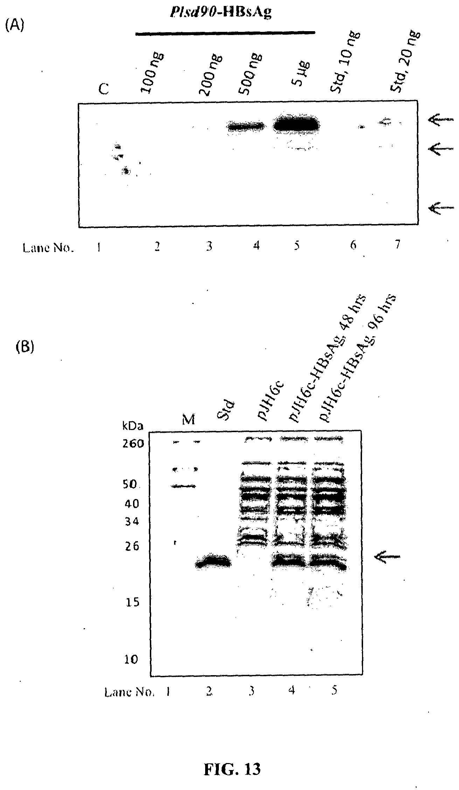

**Fig. 13** **Quantitation of the level of HBsAg expressed under the *lsd90* promoter**
   (A) Varying amounts of WCE from cultures expressing pJH6c-HBsAg for 48 hrs along with indicated amounts of purified recombinant HBsAg protein were subjected to Western blotting with antibody against anti HBsAg antibody. (B) 5 µg of WCE of cultures of cells expressing vector pJH6c (lane 3) and vector pJH6c-HBsAg after 48 hrs (lane 4) and 96 hrs (lane 5) were subjected to 12% SDS-polyacrylamide gel electrophoresis followed by silver staining. Purified recombinant HBsAg protein (500 ng) served as standard (lane 2). Lane 1 shows the protein molecular weight marker. Arrows indicate different oligomeric forms of HBsAg.

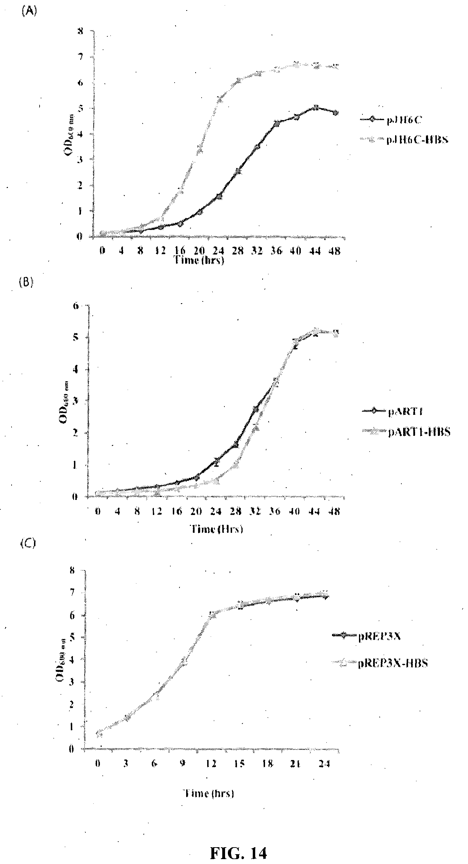

**Fig. 14** **Growth kinetics of the cultures expressing vector pJH6c-HBsAg and control vector pJH6c (A), pART1-HBsAg and control vector pART1 (B) and pREP3X-HBsAg and control vector pREP3X (C)**
   Overnight cultures of transformants of strain *SPJ25* having indicated plasmids were inoculated into the fresh medium at OD₆₀₀ 0.1 and grown further at 30°C and 200 rpm. Mean OD₆₀₀ values were plotted against time points of cultures grown in duplicate up to early stationary phase.

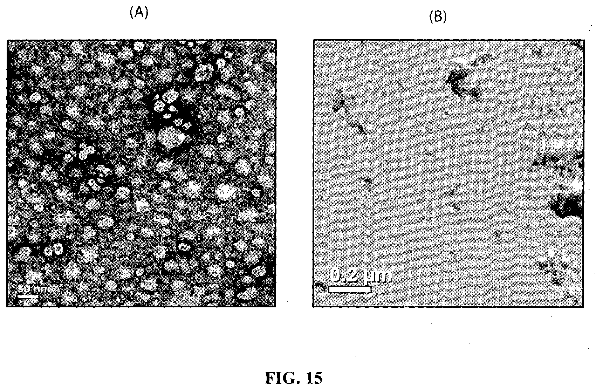

**Fig. 15** **Transmission Electron Microscopy (TEM) of purified HBsAg particles**
   (A) Cells expressing pJH6c-HBsAg show the presence of 22-nm spherical particles with a dense core, which are absent in (B) extracts of cells expressing the control vector pJH6c.

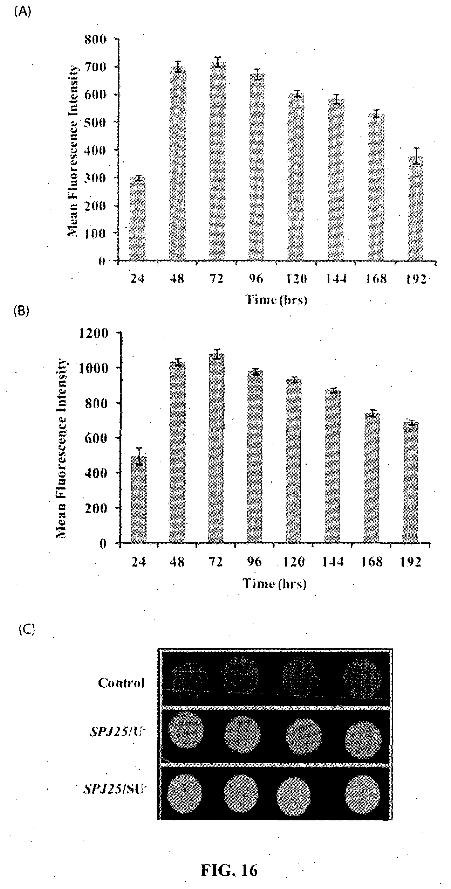

**Fig 16** **Time-course of GFP expression in wild-type strain in PMA ura⁻ medium (A, C) and medium containing sorbitol (B, C)**
   MFI cells expressing pJH6c-GFP at indicated time points of culturing in PMA ura⁻medium (A), and in PMA ura⁻ medium containing 1.2 M sorbitol (B). (C) Cultures were directly spotted on plates and photographed under long-wave length UV. U⁻ : PMA ura- and SU⁻ : PMA sorbitol-ura⁻ Control, cells expressing vector pJH6c; SPJ25: Cells expressing vector pJH6c-GFP.

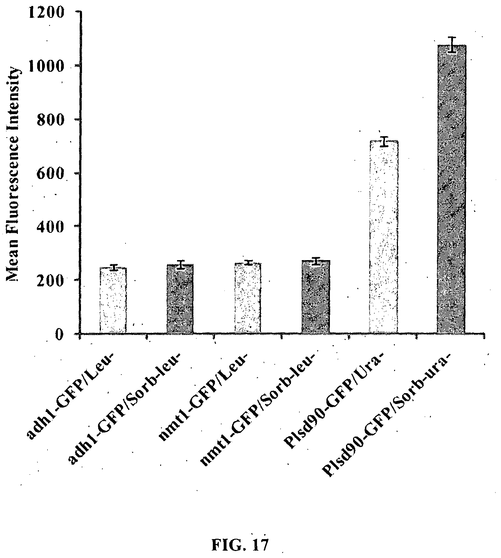

**Fig. 17** **Selective enhancement of the expression of GFP under *lsd90* promoter by sorbitol**
   Cultures of *wt* strain harbouring the indicated vectors were grown in the presence or absence of 1.2 M sorbitol and mean fluorescence intensity (MFI) at indicated time points was plotted.

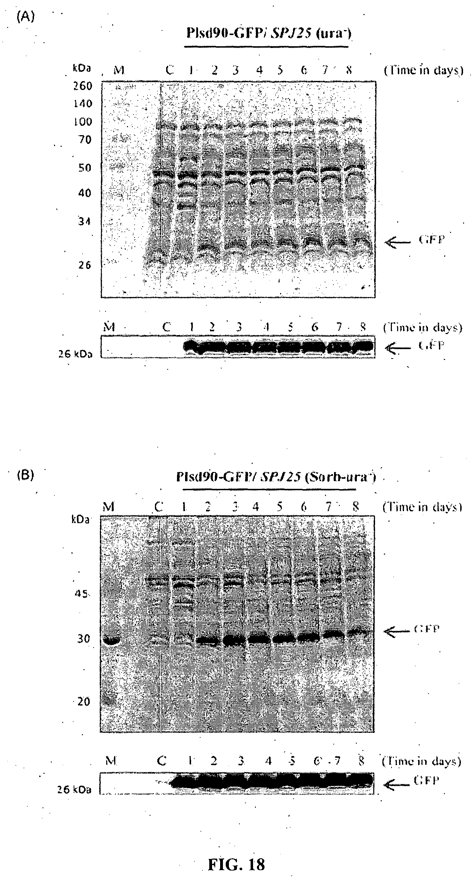

**Fig. 18** **Constitutive and stable level of expression of GFP under the new promoter *lsd90* (A) is further enhanced by growth in presence of sorbitol (B)** (Upper panel), 30 µg of protein extracts of cells expressing GFP under the promoter *lsd90* up to 8 days in PMA ura⁻ medium (A) and PMA sorb-ura- (B) were subjected to 12% SDS-PAGE followed by Coomassie staining. Lower panel (A and B) 10 µg of cell extracts were subjected to immunoblotting with anti-GFP antibody. C: protein extract of cells harboring vector pJH6c alone and M: protein molecular weight markers. GFP band of ∼28 kDa is indicated by an arrow.

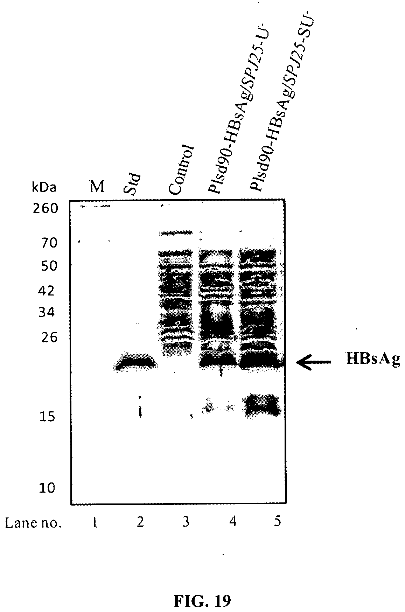

**Fig. 19** **Quantitation of the effect of sorbitol on the level of expression of HBsAg under *lsd90* promoter**
   5 µg of protein extracts of wt strain expressing Plsd90-HBsAg for 48 hrs were subjected to 12% SDS-PAGE followed by silver staining. Lane 2, purified recombinant HBsAg protein (500 ng). Protein extract of cells harbouring vector pJH6c served as control (lane 3), while lanes 4 and 5 contain extract from wild-type strain expressing HBsAg in PMA ura⁻ and in PMA ura- containing 1.2 M sorbitol, respectively. M: protein molecular weight marker, U⁻ : PMA ura⁻ and SU⁻ : PMA ura⁻ containing 1.2 M sorbitol.

**Table 1. List of primer sequences used in this study**
**Table 2. Comparison of levels of luciferase expressions using different expression systems in *S. pombe* and *P. pastoris*** Maximum level of expression achieved with *Fluc*-expression vectors pJH6c-Fluc (*lsd90*), pART1-Fluc (*adh1*), pREP3X-Fluc (*nmt1*) in *S. pombe* and pPIC3.5-Fluc (*AOX1*) in *P. pastoris* is tabulated.
**Table 3. Selective enhancement of the GFP expression under *lsd90* promoter by sorbitol.**

Comparison of GFP expression level, measured as Mean Fluorescence Intensity under different promoters in *S. pombe* in presence and absence of 1.2 M sorbitol in the medium. Data shown in Fig. 18 is tabulated.

### OBJECT OF THE INVENTION

1. The main object of the present invention is to provide a new expression vector containing a strong promoter useful for high level of expression of heterologous gene in *S. pombe*
2. Another object of the present invention is to provide a comparison of strength of new promoter with not only the existing promoters of *S. pombe,* namely the thiamine-suppressible promoter *nmt1* and the constitutive promoter *adh1,* but also the *AOX1* promoter from *P. pastoris.*
3. Still another object of the present invention is to provide a user-friendly expression vector containing a strong promoter which provides a much higher level of expression of heterologous gene in transformed *S. pombe* strain.
5. Yet another object of the present invention is to provide heterologous expression of reporter protein and a therapeutic protein, Hepatitis B surface Antigen, HBsAg, under the control of new promoter with a comparative study with other known strong promoters in *S. pombe..*
6. Yet another object of the present invention is to provide a simple culture conditions using inexpensive media for heterologous expression of proteins in S. *pombe.*
7. Still another object is to provide a process where further yields of the heterologous proteins can be maximized.

### SUMMARY OF THE INVENTION

An expression vector, designated as pJH6c, containing a promoter consisting of 1039 bp of 5' upstream region of the *lsd90* gene of *S*. *pombe,* which is useful for high level expression of heterologous genes in transformed *S. pombe* strain.

The expression vector comprising hybrid DNA construct containing a strong promoter capable to operably link to a heterologous nucleic acid encoding a desired protein, designated as pJH6c , incorporated into a *microorganism , E coli,* deposited at MTCC-IDA (IMTECH, Chandigarh) on 22.05.2012 and provided with an accession number MTCC 5688

The invention is exemplified by linking DNA sequences of two reporter genes, namely Green Fluorescent protein (GFP) and firefly luciferase (Fluc) and a therapeutic gene, Hepatitis B surface antigen (HBsAg), in the said expression vector MTCC 5688 and transforming the *S. pombe* cells by a known method, wherein the said genes have been expressed at high level when the said transformed *S*. *pombe* cells are being cultures in standard growth media.

Further, the said transformants provide further enhanced expression of the said genes when cultured in the standard media containing 1.2M sorbitol.

Accordingly, the present invention provides an expression vector for high level production of protein in transformed *S*. *pombe* strain which comprises the following steps:
i) cloning a truncated *URA3* gene, *URA3m,* comprising the complete coding sequence of *URA3* gene (orotidine 5' monophosphate decarboxylase) along with a truncated promoter in a backbone vector pUC 19,
ii) an ARS (Autonomous replication sequence) element, *mat2P-RF,* an *nmt1* terminator sequence *Tnmt1* are also cloned into the vector developed in step (i) to get an hybrid DNA construct,
iii) the NdeI site of the DNA construct obtained at step (ii) is being destroyed by standard method and a polylinker containing an NdeI site is cloned into the DNA construct to get a vector designated as pJH5,
iv) an 1 kb region of the *lsd90* gene of *S. pombe* consisting of 1039 bp of 5' upstream region is being cloned into the vector pJH5 to get the novel vector designated as pJH6c (MTCC 5688),
v) constructing chimeric DNA by cloning DNA sequence encoding a desired protein, such as herein described, at Ndel and BamHI sites downstream of the promoter *lsd90* of in the vector obtained at step(iv),
vi) transforming *S. pombe* strain with the vector obtained at step (v) and getting high level protein by culturing the transformed strain in standard growth media.

In an embodiment of present invention the production of proteins such as *GFP and* Firefly luciferase (*Fluc*) and a therapeutic protein, Hepatitis B surface antigen, HBsAg in transformed *S. pombe* strain containing the expression vector comprising the *lsd90* promoter is remarkably high and showed continuous expression of said proteins for several days at very high levels.

In another embodiment of the present invention provides the levels of expression approximately 100 mg/liter of culture for these proteins in shake flask cultures, which is very much greater than that achieved with the other known promoters from *S. pombe,* namely, the strong regulatable promoter *nmt1* and, the as yet strongest known constitutive promoter *adh1.*

In yet another embodiment of the present invention provides expression of heterologous gene in the presence of 1.2 M sorbitol to get further elevation of expression level to 300 mg protein/liter. These levels are achieved in shake flask culture and are much higher than not only those achieved with *nmt1* and *adh1* promoters of *S. pombe* (60-180 fold) but even those achieved with the *P. pastoris* promoter *AOX1* (12-18 fold).

### DETAILED DESCRIPTION OF THE INVENTION

Because of a large number of similarities with higher eukaryotes, including humans, fission yeast *Schizosaccharomyces pombe* is thought to be a potentially ideal host for expressing human proteins of therapeutic and industrial importance However, this potential is yet to be realized because of the lack of use-friendly vectors that could be used for developing cost-effective process for expression of proteins. Thus, there is still a great need for high copy vectors with greater mitotic stability for the high level expression of heterologous proteins.

As a first step towards achieving these goals, a basic high copy ATG vector was constructed that could provide up to 200 copies per cell. This involved the cloning of a truncated *URA3* gene with its minimal promoter, *URA3m,* as a selectable marker. For achieving high copy number the *mat2P-RF,* located distally to the mat2P locus, which functions as an ARS element, was cloned into the above vector. Further, the *nmt1* terminator sequence (*Tnmt1*) was also cloned to provide proper transcription termination to the mRNAs expressed from the vector. Finally, for the cloning of gene with its own translation initiation site, an ATG containing restriction site of Nde1 was incorporated into the polylinker so that the gene of interest (*Fluc* or *HBsAg*) could be cloned using Nde1 site at the 5' end and any of the suitable sites in the polylinker at the 3' end of the gene, while the promoter could be cloned between the SphI and NdeI sites. The resulting vector is named pJH5 (Fig. 1A; Verma and Singh, manuscript under preparation).

In a preliminary study, the promoter regions of three different genes of *S. pombe* that are reported to show very high levels of expression in response to different stresses (Chen et al., 2003, Mol. Biol. Cell 14: 214-229) were screened for expression of the reporter genes encoding the Firefly luciferase (*Fluc*) and *GFP.* Of these, only one promoter was found to provide high level of expression of these reporters. This promoter, *lsd90* (SEQ ID NO: 15) corresponded to the upstream ∼1Kb region of the gene *SPBC16E9.16c*/*lsd90* (PMID: 3361210; Yokoyama et al., 2008, J. Biochem. 143: 369-375). This region was PCR amplified using *S. pombe* genomic DNA as template, a sense primer carrying SphI site (SEQ ID NO: 1) and antisense primer carrying NdeI site (SEQ ID NO: 2), and cloned into the SphI and NdeI sites of the vector pJH5 to yield the expression vector pJH6c (Fig. 1B; Accession No. MTCC 5688).

The 1653 bp Firefly (*Photinus pyralis*) luciferase gene was PCR-amplified using pGL3-basic vector (Promega Corp., Madison, WI) as template, a sense primer carrying NdeI site (SEQ ID NO: 3) and antisense primer carrying BamHI site (SEQ ID NO: 7). The PCR-amplified product (*Fluc*) with 5'-NdeI and 3'-BamHI sites was cloned at NdeI and BamHI sites of the MCS under the control of *lsd90* promoter in the vector pJH6c (Accession No. MTCC 5688) and the resulting vector was named as pJH6c-Fluc (Fig. 2A).

To express the luciferase under the control of *adh1* promoter, the *Fluc* gene was PCR-amplified using sense primer carrying PstI site (SEQ ID NO: 4) and antisense primer with BamHI site (SEQ ID NO: 7). The *Fluc* gene PCR product with 5'-Pstl and 3'-BamHI sites was cloned into the MCS at PstI and BamHI sites downstream of the *adh1* promoter in the vector pART1 (Russell, 1989, San Diego: Academic Press, Inc. Nasim. A., Young, P., and Johnson, B. F., Eds. pp. 243-71). The resulting vector was named as pART1-Fluc (Fig. 2B).

To express the luciferase under the control of *adh1* promoter, heterologous gene *Fluc* was PCR-amplified using sense primer carrying XhoI site (SEQ ID NO: 5) and antisense primer with BamHI site (SEQ ID NO: 7). The *Fluc* gene with 5'-XhoI and 3'-BamHI sites was cloned into MCS at Xhol and BamHI sites downstream of the *nmt1* promoter in the vector pREP3X (Basi et al., 1993, Gene 123: 131-136). The resulting vector was named pREP3X-Fluc (Fig. 2C).

To express luciferase gene under the control of *AOX1* promoter, *Fluc* gene was PCR-amplified using sense and antisense primers carrying 5'- BamHI sites (SEQ ID NO: 6 and 7) and cloned into the MCS at BamHI site in the vector pPIC3.5 (Invitrogen, USA) downstream of the methanol-inducible *AOX1* promoter in *Pichia pastoris.* The resulting vector was named pPIC3.5-Fluc (Fig. 2D).

The *Fluc* containing vectors (pJH6c-Fluc, pART1-Fluc and pREP3X-Fluc) and the respective control vectors (pJH6c, pART1 and pREP3X) were transformed into *S*. *pombe* wild type strain *SPJ25* having *leu1-32* and *ura4D18* mutations, using lithium acetate method (Moreno *et al.,* 1991).

The *Fluc*-expression vector pPIC3.5-Fluc described above and control vector pPIC3.5 were linearized with Sall and transformed into *Pichia pastoris* host strain *GS115* (His⁻ Mut⁺ phenotype; Invitrogen). Transformants harbouring the plasmid. borne *HIS4* marker were selected on minimal plates lacking histidine and screened for Mut⁺ phenotype. Presence of the *Fluc* insert in these transformants was verified by PCR analysis using *Fluc*-specific primers (SEQ ID NO: 6 and 7).

A culture of strain *SPJ25* harbouring plasmid construct pJH6c-Fluc, expressing the *Fluc* gene under the control of *lsd90* promoter, was grown overnight in PMA ura⁻medium at 30°C and 200 rpm. The overnight culture was inoculated into fresh PMA ura⁻ medium and grown further upto 96 hrs at 30°C and 200 rpm. Culture harbouring control vector pJH6c (Accession No. MTCC 5688) was also grown in parallel. From these cultures, aliquots were harvested at intervals of 16 hrs and subjected to protein extraction. 100 ng total protein of each sample was taken to determine the luciferase activity using Luciferase Assay System (Promega, USA). All measurements were performed with GloMax^{™} 20/20 luminometer in triplicate and average luminescence values were plotted against time points (Fig. 3A).

Luciferase expression kinetics with pJH6C-Fluc was recorded upto 96 hrs. Expression level of luciferase showed an increase with cell density upto early stationary phase (48 hrs) with luciferase level of 2.4 x 10⁻¹⁴ moles (∼ 2.4 ng)/100ng protein. At later time points, expression level remained almost constant with a slight increase at 64 hrs [2.5 x 10⁻¹⁴ moles (∼ 2.5 ng)/100 ng protein]. Thus, overall the plasmid pJH6c-Fluc exhibited constitutive expression of luciferase (Fig. 3A).

For a comparative study of promoter strength, culture of strain *SPJ25* harbouring the plasmid construct pART1-Fluc, expressing the *Fluc* gene under the control of constitutive *adh1* promoter, was grown overnight in PMA leu- medium at 30°C and 200 rpm. This overnight culture was inoculated into fresh PMA leu- medium and grown further upto 96 hrs at 30°C and 200 rpm. A culture harbouring the control vector pART1 was grown in parallel. From these cultures, aliquots were harvested at intervals of 16 hrs and subjected to protein extraction. Luciferase activity was determined in triplicate with 100 ng of cell extract as described above and average luminescence values were plotted against time points (Fig. 3B).

Luciferase expression under the control of *adh1* promoter showed an increase upto log phase of growth with maximum level of 6.4 x10⁻¹⁶ moles (∼ 0.064 ng)/100 ng protein after 32 hrs of growth. A slight decline in expression level (5.1 x10⁻¹⁶ moles)/100ng protein was observed at 96 hrs of growth, probably due to decrease in growth rate with nutrient depletion (Fig. 3B).

For a comparative analysis of the promoter strength, luciferase expression was also studied under the *nmt1* promoter. Transformants of strain *SPJ25* harbouring the expression vector pREP3X-Fluc were grown overnight in PMA leu⁻ medium (supplemented with 25 µm thiamine) at 30°C with shaking at 200 rpm. The overnight culture was inoculated into fresh PMA leu⁻ medium (supplemented with 25 µm thiamine) to OD₆₀₀ 0.1 and grown further up to mid-log phase (OD₆₀₀ 0.4-0.6) at 30°C/200 rpm. Cells were collected by centrifugation, washed with sterile PMA leu⁻, resuspended in PMA leu- and further incubated at 30°C/200 rpm up to 24 hrs of induction. Culture harbouring vector control pREP3X was also grown in parallel. From these cultures, aliquots were harvested at different time points of induction and subjected to protein extraction. Luciferase activity was determined in triplicate with 100 ng of cell extract and plotted as described above (Fig. 3C)

Maximum level of luciferase expression under *nmt1* promoter (1.3 x 10⁻¹⁵ moles; ∼ 0.13 ng)/100 ng protein was observed after 18 hrs of induction (Fig. 3C).

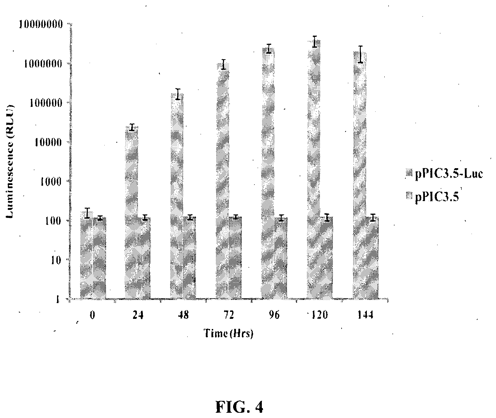

For comparison, luciferase expression was also studied under the *SOX1* promoter of *Pichia pastoris* (also referred as *P. pastoris*). A single colony of recombinant *GS115* strain harbouring the gene *Fluc,* cloned under the control of *AOX1* promoter (pPIC3.5-Fluc) was inoculated into 25 ml of BMG (Buffered Minimal Glycerol) medium in a 250 ml baffled flask and grown at 30°C in a shaking incubator (250 rpm) until the culture reached in log phase of OD₆₀₀ 2-6 (∼16-18 hrs). Cells were collected by centrifugation and resuspended in 100 ml BMM (Buffered Minimal Methanol) medium for induction in a 1 liter baffled flask and grown further at 30°C and 250 rpm. To this culture 100% methanol was added to a final concentration of 1% methanol after every 24 hrs to maintain the induction. Culture harbouring vector control pPIC3.5 was also grown in parallel. Aliquots were harvested at every 24 hrs up to 6 days of induction and subjected to protein extraction. 100 ng of cell extract of each sample was taken for luciferase assay as described above. All assays were done in triplicate and average luminescence values were plotted against time points (Fig. 4).

In cells of *P. pastoris,* expressing *Fluc* under the control of *AOX1* promoter, a continuous increase in luciferase activity was observed upto 5 days of induction with maximum luciferase level of 2.5 x 10⁻¹⁵ moles (∼ 0.25 ng)/100 ng protein. The level of luciferase decreased to almost half of the maximum expression level after 6 days of induction [1.2 x 10⁻¹⁵ moles (∼ 0.12 ng)/100ng protein] (Fig. 4).

Thus, highest level of luciferase expression was observed 2.5 x 10⁻¹⁴ moles (∼ 2.5 ng) with vector pJH6c-Fluc under the control of promoter *lsd90* (Fig. 3A). This expression level was almost 39-fold higher compared to maximum expression level achieved with the *adh1* promoter [6.4 x 10⁻¹⁶ moles (∼ 0.064 ng)/100ng protein] (Fig. 3B), almost 19-fold higher than that with the *nmt1* promoter [1.3 x 10⁻¹⁵ moles (∼ 0.13 ng)/100ng protein] (Fig. 3C) and most interestingly, almost 10-fold higher level when compared with *AOX1* promoter [2.5 x 10⁻¹⁵ moles (∼ 0.25 ng)/100ng protein] (Fig.4).

Comparative quantitation of maximum level of luciferase expression using different promoters in *S. pombe* and *P. pastoris* is tabulated in Table 2.

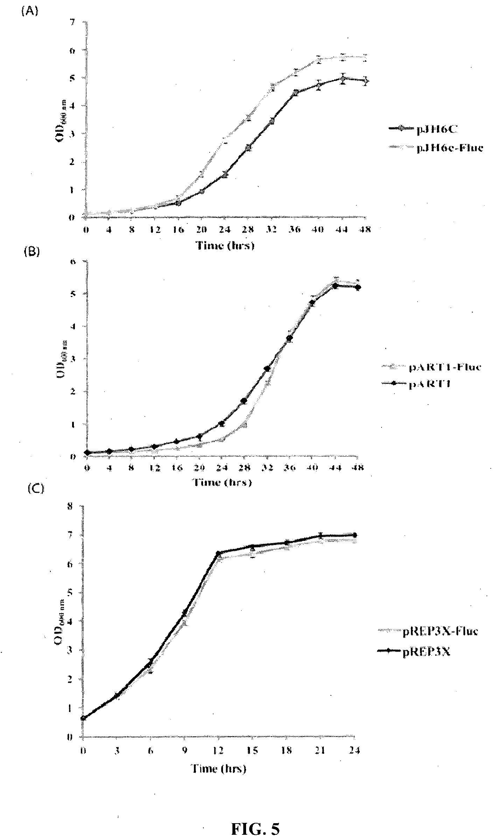

Transformants of strain *SPJ25* having plasmid pJH6c-Fluc and pJH6c (Accession No. MTCC 5688) were grown overnight in PMA ura⁻ medium at 30°C with shaking at 200 rpm. Overnight cultures were inoculated into fresh PMA ura⁻ medium at OD₆₀₀ 0.1 and grown further upto 48 hrs at 30°C with 200 rpm. For growth kinetics average OD₆₀₀ values were plotted against time course-up to early stationary phase (48 hrs). Interestingly, the cells expressing *Fluc* under the *lsd90* promoter (vector pJH6c-Fluc) had faster growth kinetics and achieved higher cell density when compared to cells expressing the empty vector (Fig. 5A).

Transformants of strain *SPJ25* having plasmid pART1-Fluc and pART1 were grown overnight in PMA leu- medium at 30°C with 200 rpm. Overnight cultures were inoculated into the fresh PMA leu⁻ medium at OD₆₀₀ 0.1 and grown further upto 48 hrs at 30°C and 200 rpm to monitor growth kinetics, as described above. Both the cultures showed almost similar growth kinetics (Fig. 5B).

As above, a culture of strain *SPJ25* harbouring *Fluc* expression vector pREP3X-Fluc was grown overnight in PMA leu⁻ medium (supplemented with 25 µm thiamine) at 30°C and 200 rpm. The overnight culture was inoculated into fresh PMA leu⁻medium (supplemented with 25 µm thiamine) to OD₆₀₀ 0.1 and grown further upto mid-log phase (OD₆₀₀ 0.4-0.6) at 30°C/200 rpm. Cells were collected by centrifugation, washed with sterile PMA leu⁻, resuspended in PMA leu⁻ and further incubated at 30°C/200 rpm for 24 hrs of induction. Culture harbouring vector control pREP3X was also grown in parallel. From these cultures, aliquots were withdrawn at different time points to study growth kinetics by measuring average OD₆₀₀ of the cultures grown in duplicate. Both the cultures showed similar growth kinetics (Fig. 5C).

To construct the *S. pombe* plasmids containing *GFP* reporter, 720 bp long *GFP* gene of *Aequorea victoria* was PCR-amplified using pGFP vector (Clontech, USA) as a template and plasmid specific primers for cloning.

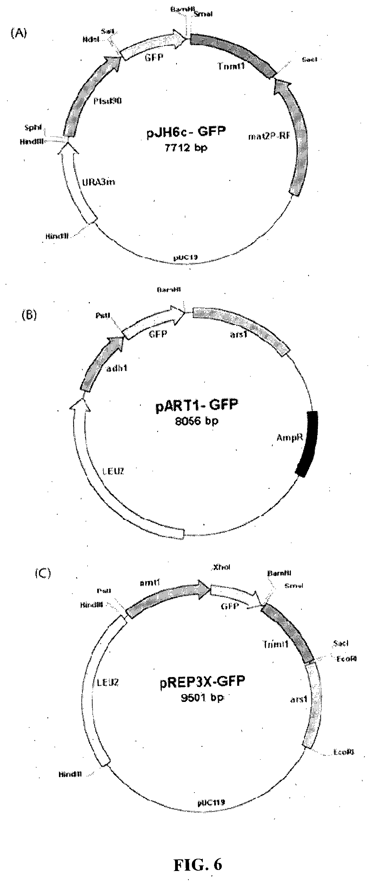

*GFP* gene was PCR-amplified using sense primer carrying Sall site (SEQ ID NO: 8) and antisense primer carrying BamHI site (SEQ ID NO: 11) and cloned at Sall and BamHI sites of the MCS under promoter *lsd90* in the vector pJH6c (Accession No. MTCC 5688). The resulting vector was named as pJH6c-GFP (Fig. 6A).

To express GFP under the control of *adh1* promoter, *GFP* gene was PCR-amplified using sense primer carrying PstI site (SEQ ID NO: 9) and antisense primer with BamHI site (SEQ ID NO: 11). The *GFP* gene with 5'-PstI and 3'-BamHI sites was cloned into MCS at PstI and BamHI sites in the vector pART1 (Russell, 1989, San Diego: Academic Press, Inc. Nasim. A., Young, P., and Johnson, B. F., Eds. pp. 243-71) downstream of the *adh1* promoter *of S. pombe.* The resulting vector was named as pART1-GFP (Fig. 6B).

To express GFP under the control of *nmt1* promoter, *GFP* gene was PCR-amplified using sense primer carrying Xhol site (SEQ ID NO: 10) and antisense primer carrying BamHI site (SEQ ID NO: 11). The *GFP* PCR product with 5'-XhoI and 3'-BamHI sites was cloned into the MCS at XhoI and BamHI sites in the vector pREP3X (Basi et al., 1993, Gene 123: 131-136) downstream of the *nmt1* promoter in *S. pombe.* The resulting vector was named as pREP3X-GFP (Fig. 6C).

*GFP*-containing vectors (pJH6c-GFP, pART1-GFP and pREP3X-GFP) as described above and the control vectors (pJH6c, pART1 and pREP3X) were transformed into *S. pombe* wild type strain *SPJ25* using lithium acetate method (Moreno *et al.,* 1991). Transformants were confirmed by colony PCR using gene-specific primers (SEQ ID NO: 10 and 11) or direct visualization of fluorescence in the colonies under long-wave UV light.

To study the kinetics of GFP expression under different promoters, FACS analysis was carried out. 1 ml aliquots of cultures expressing GFP under different promoters were harvested, fixed in 2% PFA and washed with 1 ml of 1X PBS. The cell pellet was then resuspended in 500 µl of 1X PBS and kept at 4°C for further analysis. For quantitation and comparison, 50000 cells of each samples were analyzed for fluorescence intensity represented as Mean Fluorescence Intensity (MFI), using a Becton Dickinson FACsorter with standard GFP settings (excitation wavelength, 488 nm; emission filter, 515-550 nm) using FL1-H channel. To eliminate the background autofluorescence, cultures harbouring vector control were analyzed under the same conditions.

In cells expressing GFP under the control of promoter *lsd90* (Vector pJH6c-GFP) the level of GFP fluorescence showed an increase with cell density upto early stationary phase at 48 hrs (with Mean Fluorescence Intensity of 751). Thereafter, the level of GFP expression remained almost constant with a slight increase at 64 hrs (Mean Fluorescence Intensity = 780) of growth (Fig. 7A).

In contrast, time-course FACS analysis of GFP expression under control of the strong constitutive *adh1* promoter (plasmid pART1-GFP) showed maximum fluorescence in log-phase culture (32 hrs of growth) with a Mean Fluorescence Intensity (MFI) of 233 (Fig. 7B).

For a comparative analysis of promoter strength, GFP expression was also studied under the control of *nmt1* promoter, the strongest known regulatable promoter in *S. pombe.* FACS analysis showed maximum level of Mean Fluorescence Intensity (MFI) of 256 after 18 hrs of induction (Fig. 7C).

Comparison of maximum fluorescence level of GFP expressed under the promoters *lsd90, adh1* and *nmt1* by FACS analysis showed that highest level of GFP fluorescence (with 780 Mean Fluorescence Intensity) was observed with vector pJH6c-GFP having promoter *lsd90* (Fig. 7A), which was almost 3-fold higher compared to maximum expression level achieved with the vector pART1-GFP under *adh1* promoter with Mean Fluorescence Intensity of 233 (Fig. 7B) and the vector pREP3X-GFP having *nmt1* promoter with Mean Fluorescence Intensity (MFI) of 256 (Fig 7C).

For quantitation of GFP expression at protein level, whole cell extracts were prepared from cells harbouring GFP-expression vector pJH6c-GFP at different time points along with the cell extract of the culture having pJH6c (Accession No. MTCC 5688) vector alone. Cell extracts (30 µg each) were resolved by electrophoresis on a 12% SDS-polyacrylamide gel followed by staining with Coomassie blue (Fig. 8A).

Cell extracts (10 µg each) were resolved by 12% SDS-polyacrylamide gel electrophoresis and subjected to Western blotting using anti-GFP polyclonal antibody (Fig. 8B).

SDS-Polyacrylamide gel electrophoresis (Fig. 8A) and western blotting analysis (Fig. 8B) showed that GFP expressed with *lsd90* promoter (vector pJH6c-GFP) gave a strong band in the SDS-gels in extracts prepared from cultures grown for 16 hours up to 96 hours, reaching maximum level after 48 hours of culture.

Growth Kinetics of the cultures expressing GFP in *S. pombe* was studied under conditions similar to the *Fluc*-expression under the control of promoters *lsd90, adh1* and *nmt1* (Fig. 5). It was found that the growth kinetics of cells expressing the vectors pART1-GFP and pREP3X-GFP was almost similar to the cells containing only the vectors pART1 and pREP3X, respectively (Fig. 9B and 9C). Interestingly, cells expressing *GFP* under the *Lsd90* promoter (vector pJH6c-GFP) showed faster growth kinetics and achieved higher cell density when compared to cells expressing the empty vector (Fig. 9A).

Next, the level of expression of the therapeutic protein Hepatitis B surface antigen (HBsAg) was investigated. The 678 bp long *HBsAg* gene (*ayr* subtype, SEQ ID NO: 16) containing 5'-NdeI and 3'-BamHI sites was commercially synthesized (Ocimum Biosolutions, USA) based on codon choice specific for highly expressed genes in *S*. *pombe* (Russell, 1989, San Diego: Academic Press, Inc. Nasim. A., Young, P., and Johnson, B. F., Eds. pp. 243-71). This synthetic gene was provided as clone at SmaI site in the vector pEN08H (pEN08H-HBsAg; Ocimum Biosolutions, USA). For subsequent cloning, *HBsAg* gene was either excised out or PCR amplified using this vector as template.

To express HBsAg under the control of *lsd90* promoter, HBsAg gene was excised out with NdeI and BamHI from the vector pEN08H-HBsAg (Ocimum Biosolutions, USA) and cloned into the MCS of the vector pJH6c (Accession No. MTCC 5688) at Nde1 and BamHI sites downstream of the new promoter *lsd90.* The resulting vector was named pJH6c-HBsAg (Fig. 10A).

To express *HBsAg* gene under the control of the *adh1* promoter, the *HBsAg* gene was PCR-amplified from the vector pEN08H-HBsAg using sense primer containing PstI site (SEQ ID NO: 12) and antisense primer with BamHI site (SEQ ID NO: 14). *HBsAg* gene with 5'-PstI and 3'-BamHI sites was cloned into the MCS at PstI and BamHI sites in the vector pART1 (Russell, 1989, San Diego: Academic Press, Inc. Nasim. A., Young, P., and Johnson, B. F., Eds. pp. 243-71) downstream of the *adh1* promoter of *S. pombe.* The resulting vector was named as pART1-HBsAg (Fig. 10B).

To express the HBsAg gene under the control of *nmt1* promoter, the *HBsAg* was PCR-amplified from the vector pEN08H-HBsAg using sense primer containing XhoI site (SEQ ID NO: 13) and antisense primer carrying BamHI site (SEQ ID NO: *14). HBsAg* gene with 5'-XhoI and 3'-BamHI sites was cloned into the MCS at XhoI and BamHI sites in the vector pREP3X (Basi et al., 1993, Gene 123: 131-136) downstream of the *nmt1* promoter in *S. pombe.* The resulting vector was named as pREP3X-HBsAg (Fig. 10C).

*HBsAg*-containing vectors (pJH6c-HBsAg, pART1-HBsAg and pREP3X-HBsAg) as described above and the control vectors (pJH6c, pART1 and pREP3X) were transformed into *S. pombe* wild-type strain *SPJ25* (having *leu1-32* and *ura4D18* mutations) using lithium acetate method (Moreno *et al.,* 1991). Transformants were confirmed by colony PCR using gene-specific primers (SEQ ID NO: 13 and 14).

10 µg of whole cell extract prepared from cells harbouring control vector (pJH6c; Accession No. MTCC 5688 and pART1) and expressing HBsAg under the promoters *lsd90* (plasmid pJH6c-HBsAg) and *adh1* (pART1-HBsAg) at different time points were resolved by electrophoresis on 12% SDS-Polyacrylamide gel and subjected to Western blotting using anti-HBsAg polyclonal antibody (Fig 11A).

A higher amount (100 µg) of cell extract prepared from the cells having vector control pART1 and HBsAg expression vector pART1-HBsAg was used for immunoblotting as mentioned above (Fig. 11B).

A culture of strain *SPJ25* harbouring *HBsAg*-expression vector pREP3X-HBsAg was grown overnight in PMA leu- medium (supplemented with 25 µm thiamine) at 30°C and 200 rpm. The overnight culture was inoculated into fresh PMA leu⁻medium (supplemented with 25 µm thiamine) to OD₆₀₀ 0.1 and grown further upto mid-log phase (OD₆₀₀ 0.4-0.6) at 30°C/200 rpm. Cells were collected by centrifugation, washed with sterile PMA leu⁻, resuspended in PMA leu- and further incubated at 30°C/200 rpm for 24 hrs. Culture harbouring vector control pREP3X was also grown in parallel. From these cultures, aliquots were withdrawn at different time points and 100 µg protein extract of vector control and recombinant vector pREP3X-HBsAg were subjected to immunoblotting with anti-HBsAg polyclonal antibody. Purified recombinant HBsAg protein (100 ng) from *P. pastoris* was used as standard (Fig. 11C).

Significantly higher level of expression of HBsAg was achieved with *lsd90* promoter. While strong bands were detected in case of vector pJH6c-HBsAg even with 10 □g of cell extract (Fig. 11A), there were very little detectable bands in case of western blots of 10 □g of protein extracts from cells expressing HBsAg under the *adh1* promoter (Fig. 11A). Even upon loading 10 times higher protein extracts of cell expressing HBsAg under the control of *adh1* promoter (vector pART1-HBsAg; Fig. 11B) or *nmt1* promoter (vector pREP3X-HBsAg; Fig. 11C) the level of HBsAg expressed appeared much lower than that achieved with *lsd90* promoter (Fig. 11A).

For comparative quantitation, protein extracts of cells expressing HBsAg under different promoters *of S. pombe* were subjected to 12% SDS-PAGE and Western blotted with anti-HBsAg polyclonal antibody (Fig. 12). The level of HBsAg expression using *lsd90* promoter was estimated as at least 20-fold higher than that with *nmt1* promoter and 60-fold higher than that achieved with *adh1* promoter. Furthermore, a 3-fold higher level of HBsAg was observed with *nmt1* promoter as compared to *adh1* promoter (Fig. 12).

The level of HBsAg was determined by densitometry of the bands in linear range by dilutions of the culture expressing pJH6c-HBsAg at 48 hrs in comparison to the purified recombinant HBsAg protein from *P. pastoris* (10 ng and 20 ng; Prospec, USA) as standard. 5 µg of protein extract of pJH6c (Accession No. MTCC 5688) culture was taken as control (Fig 13A). 5 µg of protein extracts of cells expressing control vector pJH6c (Accession No. MTCC 5688) and vector pJH6c-HBsAg after 48 hrs and 96 hrs of growth were resolved by electrophoresis on a 12% SDS polyacrylamide gel and silver stained. Purified recombinant HBsAg protein (500 ng) from *P. pastoris* (Prospec, USA) was loaded as standard (Fig. 13B).

The level of HBsAg expression was measured by immunoblotting (Fig. 13A) and silver staining (Fig. 13B) to be at least 10% of total cellular protein. Accordingly, the total amount of HBsAg was estimated to be approximately 100 mg/l of culture (Fig. 13A and 13B).

Published levels of HBsAg expression using the *AOX1* promoter in *P. pastoris* at shake flask level are: 18.31 mg/l (Hadiji-Abbes et al., 2009, Protein Expr. Purif. 66: 131-7) and 27 mg/l (Liu et al., 2009, Appl. Biochem. Biotechnol. 158: 432-44). Thus, *lsd90* promoter provides a level of expression that is 4- to 6-fold higher than that reported with *AOX1* promoter of *P. pastoris.*

On SDS polyacrylamide gel electrophoresis, the products of HBsAg appeared in three oligomeric forms of sizes ∼ 24 kDa, 34 kDa and 40 kDa (Fig. 11 and 12). The apparent molecular weight expected for HBsAg monomer is 24 kDa and two other oligomeric forms observed here may represent the partially reduced forms of HBsAg, which are generated because of extensive cross-linking of cysteine residues as inter-molecular disulphide bonds that could not be reduced by DTT alone (Ottone *et al.,* 2007; Kim *et al.,* 2009). However, when additional reducing agent β-ME was added, almost all HBsAg protein was found in the monomeric form of ∼24 kDa (Fig. 13B).

Growth Kinetics of the cultures expressing HBsAg in *S. pombe* was studied under conditions similar to those for GFP and luciferase expression under the control of promoters *lsd90, adh1* and *nmt1* (Fig. 5 and 9). It was found that the growth kinetics of the cells expressing *HBsAg* in vectors pARTI-HBsAg and pREP3X-HBsAg was almost similar to the cells containing only the vectors pART1 and pREP3X, respectively (Fig. 14B and 14C). Interestingly, cells expressing *HBsAg* under the *lsd90* promoter (vector pJH6c-HBsAg) showed a faster growth kinetics and achieved higher cell density than cells expressing the empty vector (Fig. 14A).

Protein extract of cells expressing HBsAg under *lsd90* promoter for 48 hrs was subjected to sucrose gradient centrifugation in ultracentrifuge (Sorvall WX ULTRA series, Thermo Scientific) using Beckman 50 TI rotor at 50000 rpm for 21 h at 4°C. Fractions with high density of recombinant HBsAg were pooled and dialyzed against protein extraction buffer. The dialyzed sample was concentrated, negatively stained with uranyl acetate and examined by transmission electron microscope (JEOL JEM-2100, Japan) operating at 120 KV.

In enriched fractions particles with diameter of approximately 22 nm, which is characteristic of correctly folded functional HBsAg protein, were observed (Fig. 15A). The particles had a spherical structure with the dense core representing the characteristic central pore, similar to that of HBsAg particles isolated from HBV-infected human (Yamaguchi *et al.,* 1998). Similar particles are produced by expression of *HBsAg* gene in *S. cerevisiae* and *P. pastoris* (Yamaguchi *et al.,* 1998; Vessileva *et al.,* 2001; Vellanki *et al.,* 2007; Liu *et al.,* 2009).

Further optimization of expression was attempted by adding various reagents, like the osmotic stabilizer, heat stress and oxidative stress (H₂O₂). Significantly higher levels of GFP and HBsAg were observed when the *pombe* cells were grown in the presence of 1.2 M sorbitol (Fig. 16A and 16B).

The level of GFP expression under the control of *lsd90* promoter in wild-type strain was quantified by flow cytometry analysis. Cultures of wild-type strain were grown in minimal media, either PMA ura⁻ or in PMA sorbitol-ura- containing 1.2 M sorbitol, at 30°C and 200 rpm. Aliquots were taken at 24 hrs intervals upto 8 days for FACS analysis.

In wild-type cells, maximum level of GFP expression in PMA ura⁻ medium was achieved after 72 hrs of growth (Mean Fluorescence Intensity = 719), followed by a slight decrease at later time points (Fig. 16A).

Interestingly, a significantly higher level of GFP expression was observed when wild-type strain was grown in PMA ura⁻ medium containing 1.2 M sorbitol, suggesting that sorbitol enhanced the efficiency of the promoter *lsd90* or caused increased stability of GFP. Maximum level of GFP fluorescence (with Mean Fluorescence Intensity of 1080) was 1.5-fold greater than that achieved in absence of sorbitol. Moreover, the high MFI was observed for longer duration in the presence of sorbitol (Fig. 16B).

Cultures harboring the control vector (pJH6c; Accession No. MTCC 5688) and *GFP*-expression vector pJH6c-GFP were grown overnight in selective media (PMA ura⁻) in the presence or absence of 1.2 M sorbitol at 30°C and 200 rpm. Cultures were normalized to OD₆₀₀ 1.0 and spotted (10 µl) on respective minimal plates with selection (PMA ura⁻/PMA sorb-ura⁻). Plates were grown for 3-4 days at 30°C. Photographs of plates with transformants showing green fluorescence were taken under long-wave UV length light (Fig. 16C).

Comparatively much higher level of GFP-expression under the control of new promoter *lsd90* in *S. pombe* was observed on the plates containing 1.2 M sorbitol than normal plates (Fig. 16C).

Further, to check whether presence of sorbitol in the medium enhances the promoter efficiency in general or it causes stabilization of the heterologous protein against proteolytic degradation, FACS analysis was carried out to measure GFP expression under the constitutive *adh1* (vector pART1-GFP) and regulatable *nmt1* promoter (vector pREP3X-GFP) of *S. pombe* in presence and absence of sorbitol in the medium. Cultures of *S*. *pombe* wild-type strain harbouring the vectors pART1-GFP and pREP3X-GFP were grown in PMA leu⁻ medium with and without 1.2 M sorbitol and analyzed after 32 hrs of continuous growth (in case of *adh1* promoter) and after 18 hrs of induction (in case of *nmt1* promoter), where maximum level of GFP expression was observed (Fig. 17).

Contrary to results with *lsd90* promoter, no difference in the level of GFP fluorescence under the promoters *adh1* (with Mean Fluorescence Intensity of ∼250) and *nmt1* (with Mean Fluorescence Intensity of ∼270) was observed in absence and presence of sorbitol. Thus, sorbitol appears to enhance the efficiency of expression of only the *lsd90* promoter (Fig. 17, Table 3).

For protein analysis, 30 µg of protein extracts of cells expressing GFP under the promoter *lsd90* in PMA ura⁻ medium at 30°C and 200 rpm up to 8 days in shake flasks were subjected to 12% SDS-PAGE followed by Coomassie staining (Upper panel, Fig. 18A). In parallel, 10□g of extracts were immunoblotted with anti-GFP monoclonal antibody (Lower panel, Fig. 18A).

Densitometric analysis of Coomassie stained gels indicated that GFP constituted over 10% of total cellular protein in Coomassie stained gel (Fig. 18A). Furthermore, GFP level seemed to remain stable even after 8 days of continuous growth (Fig. 18A).

To check the effect of sorbitol on expression of GFP directly at protein level, 30 µg of protein extracts of cells expressing GFP under the promoter *lsd90* in PMA ura⁻medium with 1.2 M sorbitol at 30°C and 200 rpm upto 8 days in shake flasks were subjected to 12% SDS-PAGE followed by Coomassie staining (Upper panel, Fig. 18B). Immunoblotting was done in parallel with 10 µg extracts probed with anti-GFP monoclonal antibody (Lower panel; Fig. 18B). Interestingly, in the presence of 1.2 M sorbitol, the level of GFP expression under the *lsd90* promoter amounted to nearly 30% of total cellular protein (Upper panel; Fig. 18B). Furthermore, GFP level remained stable even after 8 days of continuous growth (Fig. 18B).

Next, effect of sorbitol on the level of expression of HBsAg under *lsd90* promoter was also measured at protein level. 5 µg of protein extracts were prepared form wild-type strain expressing HBsAg under the promoter *lsd90,* grown in medium with and without 1.2 M sorbitol for 48 hrs and subjected to 12% SDS-PAGE followed by silver staining. Protein extract of cells harbouring vector pJH6c (Accession No. MTCC 5688) was taken as control. As observed in case of GFP expression (Fig. 18B), very high level of HBsAg expression was detected in cultures grown in presence of 1.2 M sorbitol. It was estimated to be over 30% of total cellular protein in wild-type strains (Fig. 19). In absence of sorbitol the level of HBsAg was relatively lower, ∼10 % of total cellular protein (Fig. 13).

Thus, presence of sorbitol (1.2 M) caused a further three-fold increase in level of expression of GFP and HBsAg under the control of promoter *lsd90.* The total level of HBsAg expressed upon growth in the presence of 1.2 M sorbitol was estimated as 300 mg/l of culture at shake flask level, which amounts to be 12- to 18-fold higher than the levels reported with *P. pastoris* (Hadiji-Abbes et al., 2009, Protein Expr. Purif. 66: 131-7; Liu et al., 2009, Appl. Biochem. Biotechnol. 158: 432-44).

### EXAMPLES

In the present invention we report newly discovered *lsd90* promoter in *S. pombe* which compares very favorably with the currently known expression systems in yeasts and has great potential as a commercially viable and more user-friendly alternative system for expressing heterologous proteins. The following examples are given by way of illustration of the present invention and therefore should not be construed to limit the scope of the present invention.

### Example 1:

### Construction of expression vector

In the first step, we constructed the backbone of the vector that could provide up to 200 copies per cell. This involved the cloning of a truncated *URA3* gene with its minimal promoter, *URA3m,* as a selectable marker. For achieving high copy number the ARS element, *mat2P-RF,* located distally to the mat2P locus was cloned into the above vector. Further, the *nmt1* terminator sequence was also cloned to provide proper transcription termination to the mRNAs expressed from the vector. Finally, for the cloning of gene with its own translation initiation site, an ATG containing restriction site of Nde1 was incorporated into the polylinker so that the gene of interest (luciferase or HBsAg) could be cloned using Nde1 site at the 5' end and any of the suitable sites in the polylinker at the 3' end of the gene, while the promoter could be cloned between the SphI and Ndel sites (Fig. 1A; Verma and Singh, manuscript under preparation). Next, to construct the expression vector pJH6c (Accession No. MTCC 5688), ∼1kb region of the *S. pombe* gene *lsd90* (PMID: 3361210; Yokoyama et al., 2008, J. Biochem. 143: 369-375) was cloned as the promoter *lsd90* (*Plsd90*) at SphI and NdeI sites into MCS of the vector pJH5 (Fig. 1B).

### Example 2:

### Expression of Firefly luciferase (Fluc) gene by lsd90 promoter

To express *Fluc* gene under the control of *lsd90* promoter, PCR-amplified product (*Fluc*) with 5'-NdeI and 3'-BamHI sites was cloned in the vector pJH6c (Accession No. MTCC 5688) (Fig. 2A). For comparison, Firefly luciferase (*Fluc*) gene was also cloned in front of the *adh1* promoter in the vector pART1 (Fig. 2B; Russell, 1989, San Diego: Academic Press, Inc. Nasim. A., Young, P., and Johnson, B. F., Eds. pp. 243-71) the *nmt1* promoter in the vector pREP3X (Fig. 2C; Basi et al., 1993, Gene 123: 131-136). Luciferase gene was also cloned in front of *the AOXI* promoter in the vector pPIC3.5 *of P. pastoris* from Invitrogen (Fig. 2D).

Luciferase expression kinetics under the control of *lsd90* promoter (Vector pJH6C-Fluc) was determined upto 96 hrs. Expression level showed an increase with cell density upto early stationary phase (48 hrs) with luciferase level of 2.4 x 10⁻¹⁴ moles (∼ 2.4 ng)/100ng protein. At later time points, expression level remained almost constant with a slight increase at 64 hrs [2.5 x 10⁻¹⁴ moles (∼ 2.5 ng)/100ng protein]. Thus, overall the plasmid pJH6c-Fluc exhibited constitutive expression of luciferase (Fig. 3A).

A comparative analysis showed that the highest level of luciferase expression was observed 2.5 x 10⁻¹⁴ moles (∼ 2.5 ng)/100ng protein with vector pJH6c-Fluc under the control of *lsd90* promoter (Fig. 3A). This expression level was almost 39-fold higher compared to maximum expression level achieved with the *adh1* promoter [6.4 x 10⁻¹⁶ moles (∼ 0.064 ng)/100ng protein] (Fig. 3B), almost 19-fold higher than that with the *nmt1* promoter [1.3 x 10⁻¹⁵ moles (∼ 0.13 ng)/100ng protein] in S. *pombe* (Fig. 3C) and most interestingly, almost 10-fold higher level than that with *AOX1* promoter [2.5 x 10⁻¹⁵ moles (∼ 0.25 ng)/100ng protein] in *P. pastoris* (Fig.4; Table 2).

Comparison of growth kinetics was done by monitoring the optical density (OD) of the culture at 600nm (OD₆₀₀). It was found that the growth kinetics of the cells expressing luciferase (*Fluc*) in vectors pART1-Fluc and pREP3X-Fluc was similar to the cells containing only the vectors pART1 and pREP3X, respectively (Fig. 5B and Fig. 5C). Interestingly, the cells expressing luciferase (*Fluc*) under the *lsd90* promoter (vector pJH6c-Fluc) had faster growth kinetics when compared to cells expressing the vector control (Fig. 5A).

### Example 3:

### Heterologous expression of GFP by lsd90 promoter

Next we cloned *GFP* reporter under the control of the ***lsd90*** promoter yielding the vector pJH6c-GFP (Fig. 6A). For comparison, GFP gene was also cloned in front of the *adh1* promoter to yield the expression vector pART1-GFP (Fig. 6B) and *nmt1* promoter to yield the vector pREP3X-GFP (Fig. 6C).

In cells expressing GFP under the control of *lsd90* promoter (Vector pJH6c-GFP) the level of GFP fluorescence showed an increase with cell density upto early stationary phase at 48 hrs (with Mean Fluorescence Intensity of 751). Thereafter, the level of GFP expression remained almost constant with a slight increase at 64 hrs (Mean Fluorescence Intensity = 780) of growth (Fig. 7A).

Comparison of maximum fluorescence level of GFP expressed under the promoters *lsd90, adh1* and *nmt1* by FACS analysis showed that highest level of GFP fluorescence (with 780 Mean Fluorescence Intensity) observed with vector pJH6c-GFP having promoter *lsd90* (Fig. 7A) was almost 3-fold higher compared to maximum expression level achieved with the vector pART1-GFP under *adh1* promoter with Mean Fluorescence Intensity of 233 (Fig. 7B) and the vector pREP3X-GFP having *nmt1* promoter with Mean Fluorescence Intensity of 256 (Fig 7C).

SDS-Polyacrylamide gel electrophoresis (Fig. 8A) and western blotting analysis (Fig. 8B) showed that GFP expressed with *lsd90* promoter (vector pJH6c-GFP) could be detected as a major band in the SDS gels in extracts prepared from 16 hours up to 96 hours of culture, reaching maximum level (100 mg/liter of culture) after 48 hours of culture. The level of GFP band by *lsd90* promoter was estimated to constitute ∼ 10% of total cellular protein.

Growth Kinetics of cultures expressing GFP in *S. pombe* was studied under conditions similar to the *Fluc*-expression under the control of promoters *lsd90, adh1* and *nmt1* (Fig. 5). It was found that in case of vectors pART1-GFP and pREP3X-GFP the growth kinetics of the cells expressing *GFP* was almost similar to the cells containing only the vectors pART1 and pREP3X (Fig. 9B and 9C). Interestingly, the cells expressing *GFP* under the *lsd90* promoter (vector pJH6c-GFP) showed faster growth kinetics and achieved higher cell density when compared to cells expressing the empty vector (Fig. 9A).

### Example 4:

### Heterologous expression of therapeutic protein HBsAg by lsd90 promoter

We also tested the level of expression of the Hepatitis B surface antigen (HBsAg). We cloned a synthetic copy of the gene encoding HBsAg into the vector downstream of *lsd90* promoter (pJH6c-HBsAg; Fig. 10A), *adh1* promoter (pART1-HBsAg; Fig. 10B) and *nmt1* promoter (pREP3-HBsAg; Fig. 10C). These vectors were transformed into the suitable wild type strain of *S. pombe* and expression of HBsAg was monitored by SDS-polyacrylamide gel electrophoresis and Western Blotting.

Very much higher level of expression of HBsAg was achieved with *lsd90* promoter. While strong bands were detected in case of vector pJH6c-HBsAg even with 10 □g. of cell extracts (Fig. 11A), protein bands were barely detectable in western blots of 10 □g of protein extracts from cells expressing HBsAg under the *adh1* promoter (Fig. 11A). Even upon loading 10 times higher protein extracts of cell expressing HBsAg under the control of *adh1* promoter (vector pART1-HBsAg; Fig. 11B) or *nmt1* promoter (vector pJH6c-HBsAg; Fig. 11C) the level of HBsAg expressed appeared much lower than that achieved with *lsd90* promoter (Fig. 12A).

Quantitation was done by loading a known amount of recombinant HBsAg in the Western blots (Fig. 12; Fig 13A) and silver staining (Fig. 13B). The level of expression of HBsAg achieved with *lsd90* promoter was estimated to be very high (100 mg/liter of culture), which was 20-fold higher than that with *nmt1* promoter (pREP3X-HBsAg; Fig. 13A) and 60-fold higher than that with the *adh1* promoter (pART1-HBsAg; Fig 13A). Published levels of *HBsAg* expression using the *AOX1* promoter in *P. pastoris* at shake flask levels are only in the range of 18.31 mg/l (Hadiji-Abbes et al., 2009, Protein Expr. Purif. 66: 131-7) and 27 mg/l (Liu et al., 2009, Appl. Biochem. Biotechnol. 158: 432-44). Thus, *lsd90* promoter provides a level of expression that is 4- to 6-fold higher than that with *AOX1* promoter in *P*. *pastoris.*

The functional authenticity of the HBsAg expressed in *S. pombe,* under the control of the *lsd90* promoter (vector pJH6c-HBsAg), was confirmed by transmission electron microscopy. Presence of approximately 22 nm diameter particles, characteristic of correctly folded functional HBsAg protein, in purified HBsAg expressed in *S. pombe* was observed (Fig. 15A). The particles had a spherical structure with the dense core representing the characteristic central pore, similar to that of HBsAg particles isolated from HBV-infected humans (Yamaguchi *et al.,* 1998). Similar particles are produced by expression of *HBsAg* gene in *S. cerevisiae* and *P. pastoris* (Yamaguchi *et al*., 1998; Vessileva *et al*., 2001; Vellanki *et al*., 2007; Liu *et al*., 2009).

Comparison of growth kinetics was done by monitoring the optical density (OD) of the culture at 600nm (OD₆₀₀). It was found that the growth kinetics of the cells expressing the vectors pART1-HBsAg and pREP3X-HBsAg was similar to the cells containing only the vectors pART1 and pREP3X, respectively (Fig. 14B and 14C). Interestingly, the cells expressing *HBsAg* under the *lsd90* promoter (vector pJH6c-HBsAg) had a faster growth kinetics and achieved higher OD₆₀₀ at stationary phase of growth when compared to cells expressing the empty vector (Fig. 14A).

### Example 5:

### A. Enhancement of heterologous protein expression by lsd90 promoter in presence of sorbitol

Further optimization of expression was attempted by adding various reagents, like the osmotic stabilizer, heat stress and oxidative stress (H₂O₂). Significantly higher level of GFP and HBsAg were achieved when the pombe cells harboring the respective expression vectors were grown in the presence of 1.2 M sorbitol.

The level of GFP expression under the control of *lsd90* promoter in the wild-type strain was quantified by flow cytometry analysis. Cultures of wild type strain were grown in minimal media either PMA ura⁻ or in PMA sorbitol-ura⁻ containing 1.2 M sorbitol at 30°C and 200 rpm. Aliquots were taken at 24 hrs intervals upto 8 days for FACS analysis. In wild-type cells, maximum level of GFP expression in PMA ura⁻medium was achieved after 72 hrs of growth (Mean Fluorescence Intensity = 719), followed by a slight decrease at later time points (Fig. 16A). Interestingly, a significantly higher level of GFP expression was observed when wild-type strain was grown in PMA ura⁻ medium containing 1.2 M sorbitol suggesting that sorbitol enhanced the efficiency of the promoter *lsd90* or caused increased stability of GFP. The maximum level of GFP fluorescence (with Mean Fluorescence Intensity of 1080) was 1.5 fold greater than that achieved in absence of sorbitol. Moreover, high GFP fluorescence level was observed for longer time period in the presence of sorbitol (Fig. 16B).

Similarly, to optimize GFP expression, cultures harboring the control vector (pJH6c; Accession No. MTCC 5688) and GFP-expression vector pJH6c-GFP were grown overnight in selective media (PMA ura⁻) in the presence or absence of 1.2 M sorbitol at 30°C and 200 rpm. Cultures were normalized to OD₆₀₀ of 1.0 and spotted (10 µl) on respective minimal plates with selection (PMA ura⁻/PMA sorb-ura⁻). Plates were grown for 3-4 days at 30°C. Wild-type strain containing vector pJH6c (Accession No. MTCC 5688) alone was taken as control. Comparatively higher level of GFP fluorescence was observed on the plates containing 1.2 M sorbitol in the cells expressing GFP under *lsd90* promoter under long wavelength UV (Fig. 16C).

### B. Selective enhancement of the expression of GFP under lsd90 promoter by sorbitol

To check whether presence of sorbitol in the medium enhances the promoter efficiency in general or it causes stabilization of the heterologous protein against proteolytic degradation, FACS analysis of GFP expression was carried under the constitutive *adh1* (vector pART1-GFP) and regulatable *nmt1* promoter (vector pREP3X-GFP) of S. *pombe* in presence and absence of sorbitol in the medium. Cultures of *S*. *pombe* wild-type strain harbouring the vectors pART1-GFP and pREP3X-GFP were grown in PMA leu- medium with and without 1.2 M sorbitol and analyzed after 32 hrs of continuous growth (in case of *adh1* promoter) and after 18 hrs of induction (in case of *nmt1* promoter), where maximum level of GFP expression was observed (Fig. 17). Interestingly, contrary to results with *lsd90* promoter, no difference in the level of GFP fluorescence under the promoters *adh1* (with Mean Fluorescence Intensity of ∼250) and *nmt1* (with Mean Fluorescence Intensity of ∼270) was observed in absence and presence of sorbitol. Thus, sorbitol specifically enhances the efficiency of the *lsd90* promoter (Fig. 17, Table 3).

### C. Quantitation of higher level of GFP expression by lsd90 promoter in the presence of sorbitol

To check the effect of sorbitol on level of expression of GFP, 30 µg of protein extracts of cells expressing GFP under the *lsd90* promoter in PMA ura⁻ medium containing 1.2 M sorbitol at 30°C and 200 rpm upto 8 days in shake flasks were subjected to 12% SDS-PAGE followed by Coomassie staining (Upper panel, Fig. 18B). Immunoblotting was done in parallel with 10 µg extracts probed with anti-GFP monoclonal antibody (Lower panel; Fig. 18B). Interestingly, in the presence of 1.2 M sorbitol, GFP band intensity was estimated to constitute over 30% of total cellular protein (Upper panel; Fig. 18B). GFP level remained stable even after 8 days of continuous growth (Fig. 18B).

### D. Higher level of expression of HBsAg by lsd90 promoter in the presence of sorbitol

To determine whether the stimulatory effect of sorbitol *on lsd90* promoter was general or specific, 5 µg of protein extracts of wild-type strain expressing HBsAg under the *lsd90* promoter with and without 1.2 M sorbitol in the medium for 48 hrs were subjected to 12% SDS-PAGE followed by silver staining. Protein extract of cells harbouring vector pJH6c (Accession No. MTCC 5688) was taken as control. As in case of GFP expression (Fig. 18B), very high level of HBsAg expression was detected in cultures grown in presence of 1.2 M sorbitol which was estimated to be over 30% of total cellular protein in wild-type strains (Fig. 19). In absence of sorbitol the level of HBsAg was relatively lower, ∼10 % of total cellular protein (Fig. 13).

In conclusion, presence of sorbitol elicited a further three-fold increase in level of GFP and HBsAg expressed under the control of *lsd90* promoter. The total level of HBsAg upon growth in the presence of sorbitol was estimated to be 300 mg/l of culture at shake flask level, which is estimated to be 12- to 18-fold higher than the levels reported with *P. pastoris* (Hadiji-Abbes et al., 2009, Protein Expr. Purif. 66: 131-7; Liu et al., 2009, Appl. Biochem. Biotechnol. 158: 432-44).

### ADVANTAGES OF THE INVENTION

1. Present invention provides an expression vector containing new constitutive promoter *lsd90,* which appears to be the strongest among known promoters in *S. pombe.*
2. Present invention is user-friendly and provides stable and higher level of expression of heterologous proteins for several days using inexpensive medium and simple culture conditions.
3. The invented system yields levels of expression of three proteins Firefly luciferase, GFP and HBsAg that are much higher than those achieved with the *S*. *pombe* promoters, *nmt1* (∼20- fold) and *adh1* (∼60- fold).
4. This system provides comparatively higher level (∼10-fold) of luciferase expression than that achieved with *AOX1* promoter in *P. pastoris* whereas level of HBsAg expression is ∼ 4-6 fold higher than the levels reported in *P. pastoris* at shake flask level.
5. Further, addition of 1.2 M sorbitol, which is an inexpensive component, provides a further 3-fold increase in the level of expression of heterologous proteins (GFP and HBsAg), thus achieving overall much higher protein expression as compared to *S*. *pombe* promoters *nmt1* (∼60-fold) and *adh1* (∼180-fold).
6. The level of HBsAg expression with *lsd90* promoter in the presence 1.2 M sorbitol is ∼ 12-18 fold higher than reported levels of HBsAg expression under *AOX1* promoter *in P. pastoris.*
7. Maximum expression is achieved after 36-48 hours of culture, as compared to nearly 120 hours of culture with the *AOX1* promoter of *P. pastoris.*

**Table 1**

| **Primer Sequences** | | |
|---|---|---|
| (The underlined letters are restriction sites) | | |
| lsd90 For SphI | 5'-atgcgcatctgctacgctcacactcacc-3' | (SEQ ID NO: 1) |
| lsd90 Rev NdeI | 5'-atgccatatggatgatgaagaatagaagaatgt-3 | (SEQ ID NO: 2) |
| Fluc For Ndel | 5'-tcgacatatggaagacgccaaaaacataaa-3' | (SEQ ID NO: 3) |
| Fluc For PstI | 5'-ctgcagatggaagacgccaaaaacataa-3' | (SEQ ID NO: 4) |
| Fluc For XhoI | 5'-agtcctcgaggaagacgccaaaaacataaa-3' | (SEQ ID NO: 5) |
| Fluc For BamHI | 5'-atgcggatccggaagacgccaaaaacataaa-3' | (SEQ ID NO: 6) |
| Fluc Rev BamHI | 5'-tcagggatcctcacaatttggactttccgcc-3' | (SEQ ID NO: 7) |
| GFP For SalI | 5'-agtcatgtcgacatggctagtaaaggagaagaac-3' | (SEQ ID NO: 8) |
| GFP For PstI | 5'-agtcatctgcagatggctagtaaaggagaagaac-3' | (SEQ ID NO: 9) |
| GFP For XhoI | 5'-atgcctcgagatggctagtaaaggagaagaac-3' | (SEQ ID NO: 10) |
| GFP Rev BamHI | 5'-atgcggatccctatttgtatagttcatccatgc-3' | (SEQ ID NO: 11) |
| HBsAg For PstI | 5'-agtcatctgcagatggagtccaccacctcc-3' | (SEQ ID NO: 12) |
| HBsAg For Xhol | 5'-agtcctcgagatggagtccaccacctcc-3' | (SEQ ID NO: 13) |
| HBsAg Rev BamHI | 5'-agtcggatcctcaaatgtagacccaaaggc-3' | (SEQ ID NO: 14) |

**Table 2**

| **Vector** | **Promoter** | **Maximum luciferase expression level (moles/ 100ng protein)** |
|---|---|---|
| **pJH6c-Fluc** | **PIsd90** | **2**.**5 x 10⁻¹⁴** |
| pART1-Fluc | adh1 | 6.4 x 10⁻¹⁶ |
| pREP3X-Fluc | nmt1 | 1.3 x 10⁻¹⁵ |
| **pPIC3**.**5**-**Fluc** | **AOX1** | **2.5 x 10⁻¹⁵** |

**Table 3**

| **Vector** | **Promoter** | **Medium** | **Maximum Mean Fluorescent Intensity (MFI)** |
|---|---|---|---|
| pART1-GFP | adh1 | PMA leu⁻ | 249 |
| pART1-GFP | adh1 | PMA-Sorb leu⁻ | 257 |
| pREP3X-GFP | nmt1 | PMA leu⁻ | 266 |
| pREP3X-GFP | nmt1 | PMA sorb leu⁻ | 272 |
| pJH6c-GFP | Plsd90 | PMA ura⁻ | 719 |
| **pJH6c-GFP** | **Plsd90** | **PMA Sorb ura⁻** | **1080** |

## Claims

1. An expression vectorcontaining a promoter consisting of 1039 bp of 5' upstream region of the *lsd90* gene of *Schizosaccharomyces pombe,* which is deposited at MTCC-IDA (IMTECH , Chandigarh) under accession number MTCC 5688 and which is useful for high level expression of heterologous genes in transformed *S*. *pombe* strain.

2. An expression vector as claimed in claim 1, comprising a hybrid DNA construct wherein the *lsd90* promoter used is capable to link operably to a heterologous nucleic acid or a synthetic gene encoding a desired protein.

3. An expression vector as claimed in claims 1 and 2, wherein the heterologous nucleic acid sequences used to link to the *lsd90* promoter is selected from Firefly luciferase (*Fluc*) and GFP.

4. An expression vector as claimed in claims 1 and 2, wherein the synthetic gene operably linked to the *lsd90* promoter is encoding Hepatitis B surface antigen, HBsAg.

5. A method for high level production of protein in transformed *S. pombe* strain containing the expression vector as claimed in claim 1, which comprises the following steps:
i. cloning a truncated *URA3* gene, *URA3m,* comprising the complete coding sequence of *URA3* gene (orotidine 5' monophosphate decarboxylase) along with a truncated promoter in a backbone of conventional vector pUC19,
ii. an ARS (Autonomous replication sequence) element, *mat2P-RF,* an *nmt1* terminator sequence *Tnmt1* are also cloned into the vector of step (i) to get an hybrid DNA construct,
iii. the NdeI site of the DNA construct obtained at step (ii) is being destroyed by standard method and a polylinker containing an NdeI site is cloned into the DNA construct,
iv. an 1 kb region of the *lsd90* gene of *S. pombe* consisting of 1039 bp of 5' upstream region is being cloned into the vector of step iii. to get the novel vector deposited as MTCC5688,
v. constructing chimeric DNA by cloning DNA sequence encoding a desired protein, such as herein described, downstream of the promoter *lsd90* of in the vector obtained at step(iv),
vi. transforming *S. pombe* strain with the vector obtained at step (v) and getting high level protein by culturing the transformed strain in standard growth media.

6. A process as claimed in claim 5 wherein the production of proteins such as *GFP* and Firefly luciferase (*Fluc*) and a therapeutic protein, Hepatitis B surface antigen, HBsAg in transformed *S. pombe* strain containing the expression vector, comprising the *lsd90* promoter and the heterologous genes, is remarkably high and showed continuous expression of said heterologous genes for several days at very high levels such as herein described.

7. The process as claimed in claim 5, wherein the highest level of expression of luciferase which is being achieved after 48 hours of culture is 39-fold higher than that achieved with *adh1* promoter in *S. pombe* after 32 hours of culture, 19-fold higher than that achieved with *nmt1* promoter in *S. pombe* after 18 hours of induction, and 10-fold higher than that achieved with *AOX1* promoter in *P. pastoris* after 120 hours of induction.

8. A process as claimed in claim 5 in wherein maximum level of expression of *GFP* achieved is being three-fold higher than that achieved with the *nmt1* and *adh1* promoters.

9. A process as claimed in claim 8, wherein the maximum level of expression of *HBsAg* is being 60- fold higher than that achieved with *adh1* promoter and 20-fold higher than that achieved with *nmt1* promoter.

10. A process as claimed in claim 5 wherein culture of transformed *S. pombe* strains, having the expression vector as claimed in claim 1 , produced three-fold greater level of proteins encoded by *GFP* and *HBsAg* genes, when grown in the presence of 1.2 M sorbitol.

11. A process as claimed in claim 5 wherein culture of transformed *S pombe* strains having the expression vector of claim 1, grown in the presence of 1.2 M sorbitol, produced proteins encoded by *GFP* and *HBsAg* genes which comprises 30% of total cellular proteins.

12. A process as claimed in claim 5 wherein the level of expression of *GFP* under the *lsd90* promoter in presence of 1.2 M sorbitol is being estimated to be ∼300 mg/liter.

13. The process as claimed in claim 5 wherein the level of expression of *HBsAg* under the *lsd90* promoter in presence of 1.2 M sorbitol is being estimated to be ∼300 mg/liter.

## Patentansprüche

1. Expressionsvektor, der einen Promotor enthält, bestehend aus 1039 bp einer 5'-Upstream-Region des *lsd90*-Gens von *Schizosaccharomyces pombe,* das das bei MTCC-IDA (IMTECH, Chandigarh) unter Zugangsnummer MTCC 5688 hinterlegt ist und welches nützlich ist für hochgradige Expression von heterologen Genen in transformiertem *S. pombe*-Stamm.

2. Expressionsvektor nach Anspruch 1, umfassend ein Hybrid-DNA-Konstrukt, wobei der verwendete *lsd90*-Promotor in der Lage ist, operativ an eine heterologe Nukleinsäure oder ein synthetisches Gen, das für ein gewünschtes Protein codiert, zu binden.

3. Expressionsvektor nach den Ansprüchen 1 und 2, wobei die heterologen Nukleinsäuresequenzen, die zur Verknüpfung mit dem *lsd90*-Promotor verwendet werden, ausgewählt sind aus Leuchtkäfer-Luciferase *(Fluc)* und GFP.

4. Expressionsvektor nach den Ansprüchen 1 und 2, wobei das synthetische Gen, das operativ mit dem *lsd90*-Promotor verbunden ist, für Hepatitis-B-Oberflächenantigen, HBsAg codiert.

5. Verfahren zur Herstellung von Protein in transformiertem *S*. *pombe*-Stamm in großem Maßstab, enthaltend den Expressionsvektor nach Anspruch 1, das die folgenden Schritte umfasst:
i. Klonieren eines trunkierten *URA3*-Gens, *URA3m,* umfassend die vollständige codierende Sequenz des *URA3*-Gens (Orotidin-5'-monophosphat-Decarboxylase) zusammen mit einem trunkierten Promotor im Backbone eines herkömmlichen Vektors pUC 19,
ii. ein ARS-Element (autonome Replikationssequenz), *mat2P-RF,* eine *nmt1-*Terminatorsequenz *Tnmt1* werden ebenfalls in den Vektor von Schritt (i) cloniert, um ein Hybrid-DNA-Konstrukt zu erhalten,
iii. die Ndel-Stelle des in Schritt (ii) erhaltenen DNA-Konstrukts wird durch Standardverfahren zerstört und ein Polylinker enthaltend eine Ndel-Stelle wird in das DNA-Konstrukt cloniert,
iv. eine 1 kb-Region des *lsd90*-Gens von *S*. *pombe,* bestehend aus 1039 bp von 5'-Upstream-Region wird in den Vektor von Schritt iii. cloniert, um den neuen Vektor zu erhalten, der als MTCC5688 hinterlegt ist,
v. Konstruieren von chimärer DNA durch Klonen von DNA-Sequenz codierend für ein gewünschtes Protein, wie hierin beschrieben, downstream des in dem in Schritt (iv) erhaltenen Promotors *lsd90,*
vi. Transformieren von *S*. *pombe*-Stamm mit dem in Schritt (v) erhaltenen Vektor und Erhalten eines Proteins in großem Maßstab durch Kultivieren des transformierten Stamms in Standardwachstumsmedien.

6. Verfahren nach Anspruch 5, wobei die Produktion von Proteinen wie *GFP* und Leuchtkäfer-Luciferase (*Fluc*) und einem therapeutischen Protein, Hepatitis-B-Oberflächenantigen, HBsAg in transformiertem *S*. *pombe*-Stamm, enthaltend den Expressionsvektor, umfassend den *lsd90*-Promotor und die heterologen Gene, bemerkenswert hoch ist und eine kontinuierliche Expression der heterologen Gene für mehrere Tage auf sehr hohen Niveaus, wie hierin beschrieben, zeigte.

7. Verfahren nach Anspruch 5, wobei der höchste Grad an Expression von Luciferase, der nach 48 Stunden Kultivierung erreicht wird, um das 39-fache höher ist als der, der mit dem *adh1*-Promotor in *S*. *pombe* nach 32 Stunden Kultivierung erreicht wird, um das 19-fache höher ist als der, der mit *nmt1*-Promotor in *S*. *pombe* nach 18 Stunden Induktion erreicht wird, und um das 10-fache höher ist als der, der mit *AOX1-*Promotor in *P. pastoris* nach 120 Stunden Induktion erreicht wird.

8. Verfahren nach Anspruch 5, wobei der maximale erreichte Expressionsgrad von *GFP* um das 3-fache höher ist als derjenige, der mit den *nmt1-* und *adh1*-Promotoren erreicht wird.

9. Verfahren nach Anspruch 8, wobei der maximale Expressionsgrad von *HbsAg* um das 60-fache höher ist als der, der mit dem *adh1*-Promotor erreicht wird, und um das 20-fache höher ist als der, der mit dem *nmt1*-Promotor erreicht wird.

10. Verfahren nach Anspruch 5, wobei Kultivierung von transformierten *S*. *pombe-*Stämmen mit dem Expressionsvektor nach Anspruch 1 einen um das Dreifache größeren Gehalt von Proteinen, die für *GFP-* und *HBsAg*-Gene codieren, wenn sie in Gegenwart von 1,2 M Sorbitol gezüchtet werden.

11. Verfahren nach Anspruch 5, wobei Kultivierung von transformierten *S*. *Pombe-*Stämmen mit dem Expressionsvektor von Anspruch 1, gezüchtet in Gegenwart von 1,2 M Sorbitol, Proteine, die für *GFP-* und *HBsAg*-Gene codieren und 30% der gesamten zellulären Proteine umfassen, produzierte.

12. Verfahren nach Anspruch 5, wobei der Expressionsgrad von *GFP* unter dem *lsd90-*Promotor in Gegenwart von 1,2 M Sorbitol auf -300 mg/Liter geschätzt wird.

13. Verfahren nach Anspruch 5, wobei der Expressionsgrad von *HBsAg* unter dem *lsd90-*Promotor in Gegenwart von 1,2 M Sorbitol auf -300 mg/Liter geschätzt wird.

## Revendications

1. Vecteur d'expression contenant un promoteur constitué de 1039 pb de la région en amont en 5' du gène *lsd90* de *Schizosaccharomyces pombe,* qui est déposé à la MTCC-IDA (IMTECH, Chandigarh) sous le numéro d'accession MTCC 5688 et qui est utile pour l'expression à un taux élevé de gènes hétérologues dans la souche transformée de *S. pombe.*

2. Vecteur d'expression tel que revendiqué dans la revendication 1, comprenant une construction d'ADN hybride dans lequel le promoteur *lsd90* utilisé est capable de se lier de façon fonctionnelle à un acide nucléique hétérologue ou un gène de synthèse codant pour une protéine souhaitée.

3. Vecteur d'expression tel que revendiqué dans les revendications 1 et 2, dans lequel la séquence d'acide nucléique hétérologue utilisée pour la liaison au promoteur *lsd90* est choisie parmi la luciférase de la luciole (*Fluc*) et la GFP.

4. Vecteur d'expression tel que revendiqué dans les revendications 1 et 2, dans lequel le gène de synthèse lié de façon fonctionnelle au promoteur *lsd90* code pour l'antigène de surface de l'hépatite B, HBsAg.

5. Procédé de production à un taux élevé d'une protéine dans la souche transformée de *S. pombe* contenant le vecteur d'expression tel que revendiqué dans la revendication 1, qui comprend les étapes suivantes :
i. le clonage d'un gène *URA3* tronqué, *URA3m,* comprenant la séquence codante complète du gène *URA3* (orotidine-5'-monophosphate décarboxylase) accompagnée d'un promoteur tronqué dans un squelette de vecteur pUC19 classique,
ii. un élément d'ARS (séquence à réplication autonome), *mat2P-RF,* une séquence terminateur de *nmt1, Tnmt1,* sont également clonés dans le vecteur de l'étape (i) pour obtenir une construction d'ADN hybride,
iii. le site NdeI de la construction d'ADN obtenue à l'étape (ii) est détruit par un procédé classique et un polylieur contenant un site NdeI est cloné dans la construction d'ADN,
iv. une région de 1 kb du gène *lsd90* de *S. pombe* constituée de 1039 pb de la région en amont en 5' est clonée dans le vecteur de l'étape iii. pour obtenir le nouveau vecteur déposé en tant que MTCC5688,
v. la construction d'ADN chimérique par clonage de la séquence d'ADN codant pour une protéine souhaitée, telle que décrite ici, en aval du promoteur *lsd90* dans le vecteur obtenu à l'étape (iv),
vi. la transformation de la souche de *S. pombe* avec le vecteur obtenu à l'étape (v) et l'obtention d'une protéine à un taux élevé par la culture de la souche transformée dans un milieu de croissance classique.

6. Procédé tel que revendiqué dans la revendication 5 dans lequel la production de protéines telles que la *GFP* et la luciférase de luciole (*Fluc*) et d'une protéine thérapeutique, l'antigène de surface de l'hépatite B, HBsAg, dans la souche transformée de *S. pombe* contenant le vecteur d'expression, comprenant le promoteur *lsd90* et les gènes hétérologues, est remarquablement élevée et a montré une expression continue desdits gènes hétérologues pendant plusieurs jours à des taux très élevés tels que décrits ici.

7. Procédé tel que revendiqué dans la revendication 5, dans lequel le taux le plus élevé d'expression de la luciférase qui est obtenu après 48 heures de culture est 39 fois supérieur à celui obtenu avec le promoteur *adh1* dans *S. pombe* après 32 heures de culture, 19 fois supérieur à celui obtenu avec le promoteur *nmt1* dans *S. pombe* après 18 heures d'induction, et 10 fois supérieur à celui obtenu avec le promoteur *AOX1* dans *P. pastoris* après 120 heures d'induction.

8. Procédé tel que revendiqué dans la revendication 5, dans lequel le taux maximal d'expression de la GFP obtenu est trois fois supérieur à celui obtenu avec les promoteurs *nmt1* et *adh1.*

9. Procédé tel que revendiqué dans la revendication 8, dans lequel le taux maximal d'expression de HBsAg est 60 fois supérieur à celui obtenu avec le promoteur *adh1* et 20 fois supérieur à celui obtenu avec le promoteur *nmt1.*

10. Procédé tel que revendiqué dans la revendication 5, dans lequel la culture des souches transformées de *S. pombe,* comportant le vecteur d'expression tel que revendiqué dans la revendication 1, a produit un taux trois fois supérieur des protéines codées par les gènes *GFP* et *HBsAg,* lors d'un développement en présence de 1,2 M de sorbitol.

11. Procédé tel que revendiqué dans la revendication 5, dans lequel la culture des souches transformées de *S. pombe,* comportant le vecteur d'expression selon la revendication 1, développées en présence de 1,2 M de sorbitol, a produit les protéines codées par les gènes *GFP* et *HBsAg,* lesquelles composent 30 % des protéines cellulaires totales.

12. Procédé tel que revendiqué dans la revendication 5 dans lequel le taux d'expression de la *GFP* sous le promoteur *lds90* en présence de 1,2 M de sorbitol est estimé être de ∼300 mg/litre.

13. Procédé tel que revendiqué dans la revendication 5 dans lequel le taux d'expression de *HBsAg* sous le promoteur *lds90* en présence de 1,2 M de sorbitol est estimé être de ∼300 mg/litre.
